# EUROPEAN PATENT APPLICATION

(11) **EP 4 726 034 A1**
(43) Date of publication of application: **15.04.2026**
(21) Application number: 24383088.2
(22) Date of filing: 08.10.2024
(51) Int. Cl.: C12N 9/22, C12N 15/10, C12N 15/113

(54) **CRISPR RIBONUCLEOPROTEIN COMPLEX WITH A TYPE II-B CAS9 AND THE USE THEREOF**

(71) Applicant: Consejo Superior De Investigaciones Científicas (CSIC), 28006 Madrid (ES)
(72) Inventor: González Acinas, Silvia, Barcelona (ES); Cerón Madrigal, Julián, L'Hospitalet de Llobregat (ES)
(74) Representative: Pons IP

(57) **Abstract**

The invention relates to a ribonucleoprotein complex comprising a nuclease characterized as CRISPR-associated protein of the type II-B having a sequence identity of at least 70% to SEQ ID NO: 1, and a dual guide RNA comprising a CRISPR RNA and a transactivating crRNA, or single guide RNA having a sequence identity of at least 70% to SEQ ID NO: 5. Likewise, the present invention also relates to the use of said ribonucleoprotein to bind, modify or alter target sequences in the genome of a cell or organism, or to modify the gene expression of a cell.

## Description

The invention relates to a ribonucleoprotein complex. The ribonucleoprotein complex (RNP) comprising: a nuclease characterized as CRISPR-associated (Cas) protein of the type II-B, and a guide RNA of the present invention to bind, modify or alter target sequences in the genome of a cell or organism, or to modify the gene expression of a cell. Thus, the present invention is within the molecular biology field.

### BACKGROUND ART

Recombinant DNA technology has made it possible to insert DNA sequences at targeted genomic locations and/or modify specific endogenous chromosomal sequences. Site-specific integration techniques, which employ site-specific recombination systems, as well as other types of recombination technologies, have been used to generate targeted insertions of genes of interest in a variety of organisms. Genome-editing techniques such as designer zinc finger nucleases (ZFNs), transcription activator-like effector nucleases (TALENs), or homing meganucleases, are available for producing targeted genome perturbations, but these systems tend to have low specificity and employ designed nucleases that need to be redesigned for each target site, which renders them costly and time-consuming to prepare.

Newer technologies utilizing archaeal or bacterial adaptive immunity systems have been identified, called CRISPR (Clustered Regularly Interspaced Short Palindromic Repeats), which comprise different domains of effector proteins that encompass a variety of activities (DNA recognition, binding, and optionally cleavage).

CRISPR-Cas systems include Cas proteins, which are involved in acquisition, targeting and cleavage of foreign DNA or RNA, and a guide RNA(s), which includes a segment that binds Cas proteins and a segment that binds to a target nucleic acid. The programmable nature of these systems has facilitated their use as a versatile technology for use in modification of target nucleic acid.

Despite the identification and characterization of some of these systems, expansion of the Cas9 nucleases toolbox with distinct enzymes presenting different properties is desirable to use the optimal Cas9 nuclease for each application of CRISPR-Cas technology.

### DESCRIPTION OF THE INVENTION

The authors of the present invention have developed a ribonucleoprotein complex comprising a guide RNA and an enzyme. The enzyme was isolated from deep ocean uncultured bacterium (bacteria not isolated in pure culture in laboratory conditions), identified by analysing deep ocean microbial metagenomes, with surprising distinctive biophysics properties, and structural and functional features. The isolated enzyme is a Cas9 nuclease that presents activity in a wide temperature range (5°C to 45.5°C) and a high degree of specificity, which can be higher than the canonical SpCas9.

Likewise, the present invention also discloses the guide RNA specific for said nuclease and the ribonucleoprotein complex comprising thereof (Examples 2 and 3). The inventors of the present invention have observed that the nuclease, guide RNA, and ribonucleoprotein complex disclosed herein, (i) provide activity in genome editing, such as editing of a target site on a polynucleotide sequence in a temperature range between 5°C and 45.5°C, and (ii) it is denaturalized above 45.5°C, losing its activity.

As can be seen, the examples of the present disclosure show the capacity of nuclease, guide RNA and ribonucleoprotein complex comprising them, of modifying a target nucleic acid sequence, specifically, cutting target strand and non-target strand in a PAM - dependent manner in human cell lines, *in vitro* and *in vivo* (Examples 4, 5, 8, 9 and 10) and also binds to ssDNA as other Cas9 nucleases.

After analysing the isolated Cas9 nuclease (also called "DeepOcean 2 Cas9" or "DO2 Cas9"), the inventors have discovered that:
- DO2 Cas9 is a Class II Type II-B Cas9 (1416 aa), which is the less frequent Class II Type II subtype, 213 aa smaller than FnCas9 (1629 aa) canonical Class II Type II-B Cas9 from *Francisella Novicida,* and therefore more convenient for delivery systems. FnCas9 and DO2 Cas9 present only 22.9% of similarity at amino acid level.
- Coverage of metagenomes sequences, upstream and downstream of the DO2 Cas9 gene, allowed the identification of crRNAs (repeats of the CRISPR system) and tracrRNA to infer the sequence for the guide RNA (gRNA).
- Upon in vitro translation (lVT), DO2 Cas9 showed high cleavage activity on plasmids with specific PAM sequences. Sequencing of the cleaved plasmids determined the PAM sequence for DO2 Cas9 as NGG (N= any base, G= Guanine), and the cut pattern leaves overhands of 2 nucleotides predominantly. Thus, whereas FnCas9 overhangs are predominantly 4-nt long, DO2 Cas9 leaves overhangs predominantly 2-nt long.
- DO2 Cas9 display good nuclease activity at 37°C in cultured human cells.
- Using purified protein, DO2 Cas9 protein ribonucleoprotein showed an optimal temperature range for nuclease activity in vitro between 5°C and 40.9°C, whereas the activity of its relative type II-B nuclease FnCas9 drops substantially at 5°C and 11°C.
- DO2 Cas9 would be more specific than SpCas9 and therefore would produce fewer off targets and be more accurate in diagnostic applications.
- DO2 Cas9 protein is thermosensitive. It is inactive after one hour at 35°C whereas FnCas9 is still active even after one hour at 39°C. DO2 Cas9 increases its thermostability when complexed with gRNA to form RNPs, keeping full activity after RNP is exposed to 39°C for one hour. DO2 Cas9 presents a slower denaturation transition and lower activation energy than SpCas9 and FnCas9 as observed by circular dichroism (CD) and by steady-state fluorescence spectroscopy (FS).
- DO2 Cas9 is suitable and efficient for genome editing in vivo. Using plasmids for DO2 Cas9 expression, an efficiency of around 10% was detected in the nematode *Caenorhabditis elegans* (invertebrate) and 10% in *Nicotiana benthamiana* (plant). Using mRNA for DO2 Cas9 expression, an efficiency of 50-60% was detected in zebrafish. Using DO2 Cas9 as RNP, an efficiency of 60% was detected in the nematode *Caenorhabditis elegans,* and 50% in zebrafish.
- Modeling DO2 Cas9 by Alphafold allows the identification of catalytic residues that can be mutated to produce nickase and dead versions.

In view of the foregoing, several inventive aspects have been developed which are disclosed in detailed below.

### Ribonucleoprotein complex of the invention

In one aspect, the present invention provides a ribonucleoprotein complex, hereinafter "ribonucleoprotein complex of the invention", comprising:
(i) a nuclease (hereinafter "nuclease of the ribonucleoprotein complex of the invention"), wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1; and
(ii) a guide RNA (hereinafter "guide RNA of the ribonucleoprotein complex of the invention"), wherein the guide RNA is:
   - A dual guide RNA comprising:
      a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
      b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4;
      or
- A single guide RNA comprising a nucleotide sequence having a sequence identity of at least 70%, at least 75%, at least 80%, at least 85%, at least 90%, at least 95%, at least 98%, at least 99% or 100% to SEQ ID NO: 5.

In the present invention, "ribonucleoprotein complex" refers to a guide RNA and a nuclease that are capable of forming a complex, wherein said complex can direct the nuclease to a DNA target site, enabling nuclease to recognize, bind to, and optionally nick or cleave (introduce a double-strand break) the DNA target site. These complexes play an integral part in several important biological functions that include transcription, translation and regulating gene expression and regulating the metabolism of RNA The ribonucleoprotein complex of the invention is a ribonucleoprotein that comprises a nuclease and a guide RNA. Thus, when the ribonucleoprotein complex of the invention is active, it binds and cleaves DNA. However, when the ribonucleoprotein complex is inactive, it binds to DNA but does not cut it. The ribonucleoprotein complex can be inactivated by locating and modifying the catalytic amino acid of the nuclease (Example 11) and denatured by applying heat above 45.5°C to the nuclease.

The ribonucleoprotein complex of the invention comprises a (i) nuclease and a (ii) RNA guide, wherein the nuclease comprises an amino acid sequence having a sequence identity of at least 70%, at least 75%, at least 80%, at least 5%, at least 90%, at least 95%, at least 98%, at least 99% or 100% to SEQ ID NO: 1. In a particular embodiment, the ribonucleoprotein of the invention comprises the nuclease comprising an amino acid sequence with a sequence identity of 100% to SEQ ID NO: 1.

In a more particular embodiment, the nuclease of the ribonucleoprotein complex of the invention consists of an amino acid sequence having a sequence identity of at least 70%, at least 75%, at least 80%, at least 85%, at least 90%, at least 95%, at least 98%, at least 99%, at least 99.1 %, at least 99.2%, at least 99.3%, at least 99.4%, at least 99.5%, at least 99.6%, at least 99.7%, at least 99.8%, or at least 99.9% to SEQ ID NO: 1. In another even more particular embodiment, the nuclease of the ribonucleoprotein complex of the invention consists of an amino acid sequence with a sequence identity of 100% to SEQ ID NO: 1.

SEQ ID NO: 1:
MTDSKIISPIAIDLGGLYTGLYMAQYPYGESLAQHHGKLTTLVMPEEGGKMT
WSQAARTATRHRIRSNKRRKLAKRLLKVIFTQGLSYKLNDAELEALNGLLNR
RGYNRVETDIDRQPLETVPPSWIANEFPEFFDSSSPISEQLEELLEDIPKLRD
LAADEKFNLNNREAKKQLFTDAEDEQADILKAYGILRESVTELLNSIDFGHQ
HRRKYLEAVGKEILKDSRLAELRAKLGASALKNLIGNISNLQLRALRWYFND
TAMKSADYYDGERFKSVYVRWLQNWHVDEETRVNRNNALKVIHPSEDILT

ALMTIDAELTIPPYENQNNRRPPKDQTLWLNPQSLNEQYGSQWRVWVNNL
RKRNPELAEGIAPVLEFTDRRSRLKTNKQRLTDTSLYADSYFLQRLLDRSRI
HDTYALRAISKNFKSEKLEKFRWNLKQDLGDQHTEKFLDFTQKYYQEVERA
KSGIWEQESASLLEKADINPPSKRKILNRLVGNILGVEMSAEELTTFLSDVW
TKKVHGPSTVKSICKAAEDLRKQNGNLFNMYIQSVRYRASVEAGAEKKFKG
LDKEIWQLLVKVEKAADVISGSISATETQKQRFSNTFSLAQLYTLIETDRNGF
SKVSAAAHMELAWRMTMIPVDDKEAARCSRLPADSSRPFDGVLRRLLERQ
AYEIATLKSGQIKESGVRDSEISIPVVVEENRFTFSIGLADVKKDGSKRKKLL
SLGEKQLEKWQEKNERIRQGGSGLCPYKGTILDKDAELDHIIPRSYSRDAS
GTIFNSEANLIYASRRGNQEKKDQRYGISDLAPNYLKAVYGTSDSAEITRIIE
ASVASIKKDFLFSELEGDQQRDVRHALFLERDSKAFEKVFRRLATQQSARV
NGTQAWLARKLISVLKSQLKSWLSETGNTIVFNLCKADAQQTSFVRTGLAV
TKPETTKGERQGVGSHAIDAYCAFSVAAAEGMHETLAQGAGLMEDTEWLA
TALPVDVDVNWVKRLPLYRKSELSTRPIFKEGIYAESFLTTWISEKGIESGFS
LGSNSVQWQGKDTRGLLELLAPFATKNLNVDTALASPRPVQLDISKEKAFK
HFEMVAKAPHSEVDLHQADILDALRYCTAKKDISSRLIDVQKKTLKKPEEVL
KAKDIYVKVDVDVKGLGKLKGKVTHPAIEQWKRLFLIPDIETRIGEKAVGINW
QQIGEKYFGEGSSRPHSKTRRVYSLPIVDSPDGGYRIKRIAPDGSTIWQLTS
IKDTGSSGFTVNDKGVNWKQPVAIPALRDSSVVGVGERYQPVPEQQVYFD
DWKCVDITDERVLSVSVAPGTKDRRYVSVSQTLSDFKAWLTESGIKLHSPF
EIPSRVKVDAAKFQSAHGIDVLGKPKSNLFISKVGDVVNYWYIRESSPVVIN
QYYDKALLAEREAVNETEPCDI

The term of "nuclease" is used herein to mean an enzyme which possesses catalytic activity for nucleic acid cleavage [e.g., ribonuclease activity (ribonucleic acid (RNA) cleavage), or deoxyribonuclease activity (deoxyribonucleic acid (DNA) cleavage), etc.]. Nucleases are called endonucleases if they are capable of cutting the phosphodiester bond in nucleotides located within the nucleic acid chain, and they are called exonucleases if they cut the phosphodiester bonds of the nucleotides at the ends of the chains.

In the present invention, by "cleavage" or "cut" is meant the breakage of the covalent backbone of a target nucleic acid molecule (e.g., RNA, DNA). Cleavage can be initiated by a variety of methods including, but not limited to, enzymatic or chemical hydrolysis of a phosphodiester bond. Both single-stranded cleavage and double-stranded cleavage (target strand and non-target strand) are possible. The double-stranded cleavage can occur as a result of two distinct single-stranded cleavage events.

The nuclease of the ribonucleoprotein complex of the invention comprises an amino acid sequence having a sequence identity of, at least, 70% to SEQ ID NO: 1. As use herein, the expression "sequence identity" refers to the extent to which two sequences (nucleotide or amino acid) have the same residue at the same positions in an alignment. For example, "an amino acid sequence has a sequence identity of X% to SEQ ID NO: Y" refers to the X% of residues that are identical between respective positions of two sequences when the two sequences are aligned for maximum sequence identity. The % identity is calculated by dividing the total number of the aligned residues by the number of the residues that are identical between the respective positions of the at least two sequences and multiplying by 100. Generally, computer programs can be employed for such calculations. Examples of Illustrative programs that compare and align pairs of sequences, include, but are not limited to, ALIGN, gapped BLAST, BLASTP, BLASTN, T-COFFEE, MUSCLE, MAFFT, etc.

As explained in the beginning of the present disclosure, the nuclease of the of the ribonucleoprotein complex of the invention was isolated from deep ocean uncultured bacteria.

The nuclease of the ribonucleoprotein complex of the invention is a Cas protein. The term "Cas" refers herein to a polypeptide encoded by a Cas (CRISPR- associated) gene. "CRISPR" (Clustered Regularly Interspaced Short Palindromic Repeats) loci refers to certain genetic loci encoding components of DNA cleavage systems, for example, used by bacterial and archaeal cells to destroy foreign DNA. A CRISPR locus can consist of a CRISPR array, comprising short direct repeats (CRISPR repeats) separated by short variable DNA sequences (called spacers), which can be flanked by diverse Cas (CRISPR-associated) genes. In another particular embodiment, alone or in combination with the above particular embodiments of the ribonucleoprotein of the invention, the nuclease is a Cas9 protein.

As used herein, a "Cas9 protein" or "Cas9" is a Cas endonuclease that forms a ribonucleoprotein complex (RNP) with a CRISPR RNA (crRNA) and a transactivating crRNA (tracrRNA) for specifically recognizing and cleaving all or part of a DNA target sequence. Cas9 recognizes a 3' GC-rich PAM sequence on the target dsDNA. A Cas9 protein comprises a RuvC nuclease with an HNH (H-N-H) nuclease adjacent to the RuvC-II domain. The RuvC nuclease and HNH nuclease each can cleave a single DNA strand at a target sequence (the concerted action of both domains leads to DNA double-strand cleavage, whereas the activity of one domain leads to a nick). In general, the RuvC domain comprises subdomains I, II and III, where domain I is located near the N-terminus of Cas9 and subdomains II and III are located in the middle of the protein, flanking the HNH domain. Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a transactivating CRISPR RNA (tracrRNA). In another example, a Cas9 can be in complex with a single guide RNA. Thus, in another particular embodiment of the ribonucleoprotein complex of the invention, alone or in combination with each and every one of the previous particular embodiments, the nuclease is a Cas9 of class II, still more preferably, class II-B.

The nuclease of the ribonucleoprotein complex of the invention is thermosensitive.

As used herein, "thermosensitivity" refers to a material easily affected by heat or a change in temperature. In the context of the present invention, the nuclease of the ribonucleoprotein complex of the invention shows is inactivated by exposing the protein to lower temperatures compared to other Cas9 nucleases such as SpCas9 and its relative type II-B FnCas9. The RNP formed by the nuclease of the invention is also more sensitive to heat than other nucleases such as SpCas9, and therefore there is a decrease in their activity at temperatures where other Cas9 nucleases such us SpCas9 are fully functional.

The nuclease of the ribonucleoprotein complex of the invention can be used within a broad temperature range of from a low temperature of 5°C to a high temperature of about 45.5°C. Since the nuclease of the ribonucleoprotein complex of the present invention can show its activity within a broad temperature range, those skilled in the art can use it under various temperature conditions in function of the intended purpose. For example, the nuclease of the ribonucleoprotein complex of the invention can carry out nucleic acid digestion under constant temperature conditions, under variable temperature conditions (within a certain range), or under predetermined or programmed temperature cycling conditions and the like. Particularly, the nuclease of the ribonucleoprotein complex of the invention may be enzymatically active, or has detectable nuclease activity, in a temperature range of from 5°C to 45.5°C. In some cases, the nuclease of the ribonucleoprotein complex of the invention is enzymatically active, or has detectable nuclease activity, in a temperature range of from 10°C to 40°C. In some cases, the nuclease of the ribonucleoprotein complex of the invention is enzymatically active, or has detectable optimal nuclease activity, in a temperature range of from 15°C to 35°C. In some cases, the nuclease of the ribonucleoprotein complex of the invention is enzymatically active, or has detectable optimal nuclease activity, in a temperature range of from 20°C to 30°C. In some cases, the nuclease of the ribonucleoprotein complex of present invention exhibits maximal enzymatic activity or has detectable nuclease activity (as defined by cleavage rate of a target nucleic acid when complexed with a guide RNA (gRNA) in a temperature range of from 5°C to 40.9°C. Thus, in another particular embodiment of the nuclease of the ribonucleoprotein complex of the invention, alone or in combination with each and every one of the above particular embodiments, the nuclease is enzymatically active (or has detectable nuclease activity) in a temperature range of from 5°C to 45.5°C, preferably 5°C to 10°C, more preferably at 5°C. In all ranges mentioned in the present description the extremes are included.

Additionally, the nuclease of the ribonucleoprotein complex of the invention may be denatured by high application of heat at temperatures over 35°C (1 hour at 35°C), thereby reducing or eliminating its nuclease activity whereas the canonical class II-B nuclease FnCas9 family is still active even after one hour at 39°C. The ribonucleoprotein complex is still active after 1 hour at 41°C, demonstrating that the stability of the nuclease of the invention is more resistant to temperature when complexed as RNP. Thus, in another particular embodiment of the nuclease of ribonucleoprotein complex of the invention, alone or in combination with each and every one of the above particular embodiments, the nuclease as ribonucleoprotein complex is enzymatically inactive in a temperature above 45.5°. In all ranges mentioned in the present description the extremes are included.

The terms "enzymatic activity", "enzymatically active" and "nuclease activity" as used herein with reference to a nuclease of the of the ribonucleoprotein complex of the invention refers to cleavage of one or both strands of a nucleic acid target by the protein when the protein is complexed with a gRNA.

The terms "target nucleic acid", "target site", "target sequence" or "target DNA" are used interchangeably herein and refer to a polynucleotide sequence in the genome (including choloroplastic and mitochondrial DNA) of a cell at which a double-strand break (or a single strand if the nuclease is engineered to present nickase activity), is induced in the cell genome by a nuclease of the invention. The target nucleic acid is the sequence to which the guide sequence of a nuclease gRNA (e.g., a dual gRNA or a single-molecule gRNA) will hybridize. For example, the target DNA sequence 5'-GAGCATATC-3' within a target nucleic acid is targeted by (or is bound by, or hybridizes with, or is complementary to) the RNA sequence 3'-GAUAUGCUC-5'. Suitable hybridization conditions include physiological conditions normally present in a cell. For a double-stranded target nucleic acid, the strand of the target nucleic acid that is complementary to and hybridizes with the gRNA is referred to as the "complementary strand" or "target strand"; while the strand of the target nucleic acid that is complementary to the "target strand" (and is therefore not complementary to the gRNA) is referred to as the "non-target strand" or "non- complementary strand."

The nuclease of the ribonucleoprotein complex of the invention may be capable of being guided by a gRNA to target single or double-stranded DNA in a protospacer adjacent motif (PAM)-dependent manner (protospacer adjacent motif). As is the case for many CRISPR endonucleases, such as Cas9, site-specific binding (and/or excision) of a double-stranded target DNA occurs at locations determined by (i) base pairing complementarity between the guide RNA and the target DNA; and (ii) a short motif PAM in the target DNA. The Cas endonuclease may not successfully recognise a target DNA sequence if the target DNA sequence is not followed by a PAM sequence. The PAM sequence can be any length, but is typically 1, 2, 3, 4, 5, 6, 7, 8, 9, 10, 11, 12, 13, 14, 15, 16, 17, 18, 19 or 20 nucleotides long. The term "binding", as used herein (e.g. with reference to an RNA -binding domain of a polypeptide, binding to a target nucleic acid, and the like), refers to a non-covalent interaction between macromolecules (e.g., between a protein and a nucleic acid; between a ribonucleoprotein complex (nuclease and guide RNA) and a target nucleic acid; and the like).

The term "protospacer adjacent motif" or "PAM" as used herein, refers to a short nucleotide sequence adjacent to a target sequence (protospacer) that is recognised (targeted) by a ribonucleoprotein complex that comprises a guide RNA and nuclease of the invention. In another particular embodiment of the ribonucleoprotein complex of the invention, alone or in combination with each and every one of the above particular embodiments, the nuclease binds and cleaves the target polynucleotide in a protospacer adjacent motif (PAM) - dependent manner. In another particular embodiment of the ribonucleoprotein complex of the invention, alone or in combination with each and every one of the above particular embodiments, the nuclease cleaves:
- a double-stranded target polynucleotide, at the +3 position from PAM in the target DNA strand and at the -5 and -6 position from PAM in the non-target strand, leaving mostly overhangs of 2 or 3 nucleotides,
   or
- a single-stranded target polynucleotide.

In another particular embodiment of the ribonucleoprotein complex of the invention, alone or in combination with each and every one of the above particular embodiments, the PAM sequence comprises the sequence NGG wherein N is any base and G is Guanine.

The ribonucleoprotein of the invention may be capable of being guided by a guide RNA to target single or double-stranded DNA in a PAM-dependent manner. Thus, the ribonucleoprotein comprises a (i) nuclease and a (ii) RNA guide, wherein the RNA guide is
- A dual guide RNA comprising:
   a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
   b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4;
   or
- A single guide RNA comprising a nucleotide sequence having a sequence identity of at least 70%, at least 75%, at least 80%, at least 85%, at least 90%, at least 95%, at least 98%, at least 99% or 100% to SEQ ID NO: 5.

As used herein, the term "guide RNA" or "gRNA", relates to a polynucleotide sequence that solely comprises ribonucleic acids, and that can form a ribonucleoprotein complex with a protein, such us the nuclease described herein, and enables the protein to recognize, optionally bind to, and optionally cleave, a DNA target site.

The guide RNA can be a double molecule (also referred to as "dual guide RNA" or "dgRNA"), or a single molecule (also referred as "single guide RNA" or "sgRNA") where tracrRNA and crRNA are linked by a few, normally 4, nucleotides. In both cases, the guide RNA comprises a crRNA and a tracrRNA sequence. As used herein, a tracrRNA (trans-acting CRISPR RNA) is a naturally existing molecule that hybridizes with a CRISPR RNA molecule (a crRNA) to form a dual guide RNA, or to form a single guide RNA when tracrRNA and crRNA are linked by 4 nucleotides.

On the one hand, the ribonucleoprotein complex of the invention comprises a dual guide RNA hereinafter "dgRNA of the ribonucleoprotein of the invention, comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

SEQ ID NO: 3, nucleotide repeat sequence of a crRNA, direction 5' to 3': XXXXXXXXXXXXXXXXXXXXG U U UCAG U UGCAGG U U U AU UGGGAU AGGC U U U GCAAC, where XXXXXXXXXXXXXXXXXXXX are the 20 nucleotides specific for the targeted region.
SEQ ID NO: 4, nucleotide sequence of a tracrRNA, direction 3' to 5':

UUUUUCUUUUUGGGGCCUACGGCUUCGUCCACGGCCUCAGUCAGUACUAAA
AUCUACUAAAGUCGAGCGGGCACCCGCUGAAGUUAGUCCGGAUAACCCUAU
CC

In a particular embodiment of the ribonucleoprotein complex of the invention, alone or in combination with each and every one of the previous particular embodiments, the nucleotide sequence of crRNA of the dgRNA consists of the sequence SEQ ID NO: 3. In a particular embodiment of the ribonucleoprotein complex of the invention, alone or in combination with each and every one of the previous particular embodiments, the nucleotide sequence of tracrRNA of dgRNA consists of the sequence SEQ ID NO: 4.

On the other hand, the ribonucleoprotein complex of the invention comprises a single guide RNA hereinafter "sgRNA of the ribonucleoprotein of the invention, comprising a nucleotide sequence having a sequence identity of at least 70%, at least 75%, at least 80%, at least 85%, at least 90%, at least 95%, at least 98%, at least 99% or 100% to SEQ ID NO: 5.

SEQ ID NO: 5, nucleotide sequence of a single guide RNA (direction 5' to 3'): XXXXXXXXXXXXXXXXXXXXGUUUCAGUUGCAGGUUUAUUGGGAUAGGGAAAC CUAUCCCAAUAGGCCUGAUUGAAGUCGCCCACGGGCGAGCUGAAAUCAUCU AAAAUCAUGACUGACUCCGGCACCUGCUUCGGCAUCCGGGGUUUUUCUUUU U, where XXXXXXXXXXXXXXXXXXXX are the 20 nucleotides specific for the targeted region.

The term "single guide RNA" or "sgRNA" relates to a synthetic fusion of two RNA molecules, a crRNA comprising a variable targeting domain (linked to a tracr mate sequence that hybridizes to a tracrRNA), fused to a tracrRNA.

In a particular embodiment of the sgRNA of the ribonucleoprotein complex of the invention, the nucleotide sequence of the sgRNA consists of the sequence SEQ ID NO: 5.

In some embodiments, there is a linker polynucleotide that connects the crRNA and tracrRNA to form the single guide RNA. The linker of a single guide RNA described in the present description is a stretch of nucleotides, it can have a length of at least 3 nucleotides. Preferably, the linker of a single guide RNA is GAAA.

In a particular embodiment of the ribonucleoprotein complex of the invention, the guide RNA recognises a target nucleic acid sequence in a single or a double-stranded polynucleotide.

In another particular embodiment of the ribonucleoprotein complex of the invention, the guide RNA described herein is substantially complementary along its length to a target DNA sequence. In another particular embodiment of the ribonucleoprotein complex of the invention, alone or in combination with each and every one of the above particular embodiments, the single or double-stranded target polynucleotide comprising the target nucleic acid sequence is cleaved by the nuclease described herein, preferably wherein said cleavage is DNA cleavage.

The target polynucleotide may further comprise a PAM sequence directly adjacent the 3' end of a protospacer sequence, in accordance with a PAM sequence provided herein. The guide RNA may be bound to or associated with the target sequence within the ribonucleoprotein complex, so that the target polynucleotide, comprising the target sequence and PAM sequence on the non-target strand, may be associated with and so form part of a ribonucleoprotein complex of the invention.

Alteration of the sequence of the RNA guide which associates with the nuclease of the invention therefore allows the nuclease to be programmed to mark or cut single or double-stranded DNA at sites complementary to the guide RNA.

### Guide RNA (gRNA) of the invention

The present invention also encompasses a guide RNA useful to lead a nuclease to specifically modify or alter target sequences in the genome of a cell or organism. This guide RNA may be a dual guide RNA or a single guide RNA.

Thus, in another aspect, the present invention relates to a dual guide RNA, hereinafter "dgRNA of the invention", comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

In a particular embodiment of the dgRNA of the invention, alone or in combination with each and every one of the previous particular embodiments, the nucleotide sequence of crRNA consists of the sequence SEQ ID NO: 3. In a particular embodiment of the dgRNA of the invention, alone or in combination with each and every one of the previous particular embodiments, the nucleotide sequence of tracrRNA consists of the sequence SEQ ID NO: 4.

In another aspect, the present invention relates to a single guide RNA, hereinafter "sgRNA of the invention", comprising a nucleotide sequence having a sequence identity of at least 70%, at least 75%, at least 80%, at least 85%, at least 90%, at least 95%, at least 98%, at least 99% or 100% to SEQ ID NO: 5.

The term "single guide RNA" or "sgRNA" relates to a synthetic fusion of two RNA molecules, a crRNA comprising a variable targeting domain of 20 nucleotides linked to a crRNA mate sequence that hybridizes to a tracrRNA, fused to a tracrRNA.

In a particular embodiment of the sgRNA of the invention, the nucleotide sequence of the sgRNA consists of the sequence SEQ ID NO: 5.

In some embodiments, there is a linker polynucleotide that connects the crRNA and tracrRNA to form the single guide RNA. The linker of a single guide RNA described in the present description is a stretch of nucleotides, it can have a length of at least 3 nucleotides. Preferably, the linker of a single guide RNA is GAAA.

The terms "sequence identity", "guide RNA", "dual guide RNA", "single guide RNA", "crRNA sequence" and "a tracrRNA sequence" have been disclosed above for the previous inventive aspect and their definitions and their particular embodiments are applicable to the present inventive aspect.

### Nuclease of the invention

The present invention also relates to the nuclease of the ribonucleoprotein complex of the invention.

Thus, in another aspect, the present invention refers to a nuclease, hereinafter "nuclease of the invention", comprising an amino acid sequence having a sequence identity of at least 70%, at least 75%, at least 80%, at least 85%, at least 90%, at least 95%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 1. In a more particular embodiment, the nuclease of the invention comprises an amino acid sequence with a sequence identity of 100% to SEQ ID NO: 1.

In an even more particular embodiment, the nuclease of the invention consists of an amino acid sequence having a sequence identity of at least 70%, at least 75%, at least 80%, at least 85%, at least 90%, at least 95%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 1. In another even more particular embodiment, the nuclease of the invention consists of an amino acid sequence with a sequence identity of 100% to SEQ ID NO: 1.

The term "sequence identity" has been defined previously in the present description. Likewise, all the particular embodiments and definitions disclose un previous paragraphs for the the nuclease of the ribonucleoprotein complex of the invention are applicable to the present inventive aspect.

As explained in the beginning of the present disclosure, the nuclease of the invention was isolated from deep ocean uncultured bacteria

The nuclease of the invention is a Cas protein, specifically a Cas9 protein, and as indicated above this kind of proteins present endonuclease activity. Thus, in a particular embodiment, alone or in combination with the above particular embodiments, the nuclease of the invention is a Cas9 protein. In another particular embodiment of the nuclease of the invention, alone or in combination with each and every one of the previous particular embodiments, the nuclease of the invention is a Cas9 of class II, still more preferably, class II-B.

The nuclease of the invention is thermosensitive. The nuclease of the invention can be used within a broad temperature range of from a low temperature of 5°C to a high temperature of about 45.5°C. Since the nuclease of the present invention can show its activity within a broad temperature range, those skilled in the art can use it under various temperature conditions in function of the intended purpose. For example, the nuclease of the invention can carry out nucleic acid digestion under constant temperature conditions, under variable temperature conditions (within a certain range), or under predetermined or programmed temperature cycling conditions and the like. Particularly, the nuclease of the invention may be enzymatically active, or has detectable nuclease activity, in a temperature range of from 5°C to 45.5°C. In some cases, the nuclease of invention is enzymatically active, or has detectable nuclease activity, in a temperature range of from 10°C to 40°C. In some cases, the nuclease of the invention is enzymatically active, or has detectable optimal nuclease activity, in a temperature range of from 15°C to 35° C. In some cases, the nuclease of the invention is enzymatically active, or has detectable optimal nuclease activity, in a temperature range of from 20°C to 30°C. In some cases, the nuclease of present invention exhibits maximal enzymatic activity or has detectable nuclease activity (as defined by cleavage rate of a target nucleic acid when complexed with a guide RNA (gRNA) in a temperature range of from 5°C to 40.9 °C. Thus, in another particular embodiment, alone or in combination with each and every one of the above particular embodiments, the nuclease is enzymatically active (or has detectable nuclease activity) in a temperature range of from 5°C to 45.5°C. In all ranges mentioned in the present description the extremes are included.

Additionally, the nuclease of the invention may be inactivated by high application of heat at temperatures over 50°C (denatured after 1 hour at 35°C), thereby reducing or eliminating its nuclease activity. Thus, in another particular embodiment of the nuclease of the invention, alone or in combination with each and every one of the above particular embodiments, the nuclease is inactive in a temperature above 50°C. In addition, the nuclease of the ribonucleoprotein complex of the invention is denatured after 1 hour at 33°C. In all intervals mentioned in the present description the extremes are included.

The term "denatured" refers to the destruction of the characteristic properties of a protein by heat, acidity, or other effect which disrupts its molecular conformation. As use herein, the nuclease of the invention is denatured by heat above 45.5°C, altering its standard three-dimensional structure, and rendering the nuclease non-functional, that is, it cannot exert its activity.

### Polynucleotide of the invention

The present invention also encompasses a polynucleotide comprising a nucleotide sequence encoding the nuclease of the invention. Thus, in another aspect, the present invention refers to a polynucleotide, hereinafter "polynucleotide of the invention", comprising a nucleotide sequence encoding the nuclease of the invention.

The terms "polynucleotide" and "nucleic acid," used interchangeably herein, refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA -RNA hybrids, or a polymer comprising purine and pyrimidine bases or other natural, chemically or biochemically modified, non-natural, or derivatized nucleotide bases.

Alternatively, in another particular embodiment of the polynucleotide of the invention, alone or in combination with each and every one of the previous particular embodiments, the polynucleotide consists of a nucleotide sequence encoding the nuclease of the invention.

In another particular embodiment of the polynucleotide of the invention, alone or in combination with each and every one of the previous particular embodiments, the nucleotide sequence has an sequence identity of, at least 70%, at least 71%, at least 72, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% to SEQ ID NO: 2. The expression "sequence identity" has been defined above in previous paragraphs. In another particular embodiment of the polynucleotide of the invention, the nucleotide sequence has a sequence identity of 100% to SEQ ID NO: 2.

SEQ ID NO: 2:
ACTGACTCTAAAATCATCTCTCCAATCGCGATTGACCTCGGTGGTCTTTATACC
GGTCTGTATATGGCGCAATATCCGTATGGAGAGAGCCTCGCTCAGCATCATGG
CAAGCTGACAACGCTGGTTATGCCAGAAGAGGGCGGCAAAATGACCTGGTCG
CAAGCGGCGCGGACTGCTACCCGGCACCGCATACGTTCAAACAAACGTAGAA
AGCTGGCGAAACGTTTACTGAAAGTGATCTTTACTCAAGGGCTGTCTTACAAGC
TGAATGATGCAGAACTTGAGGCCTTGAATGGCTTGCTGAATCGTAGGGGGTAC
AACAGAGTAGAAACGGATATTGATCGCCAACCTCTTGAGACTGTTCCTCCTTCC

TGGATTGCCAATGAGTTCCCAGAATTTTTCGATAGTAGCAGTCCTATATCTGAG
CAATTAGAAGAACTACTGGAAGACATTCCCAAACTTCGTGATTTGGCGGCTGAT
GAGAAGTTTAATCTTAATAATCGCGAAGCAAAGAAGCAGTTATTTACAGACGCA
GAAGACGAACAAGCAGATATTCTGAAAGCCTACGGTATTCTTCGGGAATCAGTA
ACAGAGTTGCTTAACAGTATCGACTTCGGGCACCAGCATCGTCGAAAATACCTT
GAAGCCGTAGGTAAAGAGATTTTGAAGGATTCCCGATTAGCAGAGCTGCGGGC
TAAGCTCGGGGCTTCCGCCCTCAAGAATCTTATTGGCAACATCAGCAACCTGC
AGTTGCGAGCGCTAAGATGGTACTTTAACGATACCGCGATGAAGAGTGCCGAT
TACTATGATGGCGAACGCTTCAAGTCGGTATATGTTCGTTGGCTGCAGAACTG
GCATGTGGACGAAGAAACGCGAGTTAATCGCAACAATGCATTAAAAGTGATTCA
TCCTTCAGAAGATATTCTCACGGCGTTGATGACGATTGATGCAGAACTGACGAT
TCCACCCTATGAAAATCAGAATAACCGTCGACCTCCAAAAGATCAGACCTTATG
GCTTAACCCCCAGTCACTGAATGAGCAATATGGCTCACAGTGGCGTGTATGGG
TTAATAATCTCCGTAAACGTAATCCGGAGCTGGCAGAGGGAATCGCGCCTGTT
CTTGAGTTCACTGACCGCCGTTCACGTTTGAAAACCAATAAGCAGCGCCTGACT
GATACGTCTTTATATGCAGACAGCTATTTTCTTCAGCGTCTTCTTGATCGCAGCA
GGATCCATGATACCTACGCACTCCGTGCGATATCAAAGAACTTCAAGTCAGAAA
AACTTGAAAAATTCCGCTGGAACCTGAAGCAGGATCTGGGAGATCAACATACA
GAAAAGTTTCTGGACTTCACTCAGAAGTATTACCAGGAAGTGGAACGCGCAAA
AAGTGGCATCTGGGAGCAGGAATCGGCATCACTGCTTGAAAAAGCGGATATTA
ACCCTCCCTCAAAGCGAAAGATTCTGAATCGATTAGTAGGGAATATTCTTGGCG
TTGAAATGAGCGCTGAAGAACTGACCACCTTTCTGTCAGACGTATGGACGAAG
AAAGTTCATGGCCCATCAACGGTAAAGAGTATCTGCAAAGCTGCCGAAGACCT
GAGAAAACAGAATGGCAACCTCTTCAATATGTATATCCAGTCTGTACGGTATCG
AGCGTCGGTCGAAGCAGGTGCAGAGAAGAAATTTAAAGGTCTGGATAAAGAAA
TTTGGCAGCTTCTGGTTAAAGTAGAGAAAGCAGCAGATGTGATTTCTGGTTCAA
TTTCTGCGACCGAAACGCAGAAACAAAGATTCAGTAATACCTTTTCACTTGCTC
AGTTGTACACCCTGATAGAGACAGACCGGAACGGATTCTCAAAGGTCAGTGCT
GCTGCACACATGGAGCTTGCATGGCGTATGACAATGATACCGGTGGACGATAA
GGAAGCCGCACGTTGTTCGCGCTTGCCAGCAGACAGTTCTCGGCCATTCGAC
GGAGTGTTAAGACGCTTGCTCGAACGTCAGGCTTATGAAATAGCGACGCTGAA
ATCGGGGCAGATAAAAGAGTCCGGTGTCAGAGACTCTGAAATAAGCATTCCAG
TGGTGGTAGAAGAAAACCGCTTTACTTTCAGTATTGGCCTTGCAGATGTAAAAA
AAGATGGATCGAAGCGAAAAAAGCTGCTATCGCTGGGTGAGAAGCAACTGGAA

AAATGGCAAGAAAAGAATGAACGCATCAGACAGGGCGGGAGTGGCCTGTGCC
CTTATAAGGGAACAATTCTGGATAAAGATGCTGAGCTAGATCACATAATTCCAC
GCTCGTACAGTCGTGATGCATCAGGGACAATCTTCAATAGCGAAGCCAATCTTA
TTTACGCCAGCCGCCGTGGCAATCAGGAAAAGAAAGACCAACGTTACGGCATT
TCTGACTTGGCACCCAATTACCTCAAGGCCGTCTATGGGACGTCAGATTCGGC
AGAGATAACCCGGATTATCGAAGCGTCTGTGGCGTCTATTAAGAAGGACTTCCT
CTTCTCAGAGCTAGAAGGTGATCAGCAAAGGGATGTCCGCCATGCCTTATTCC
TGGAACGTGATTCAAAGGCATTTGAGAAGGTATTCAGACGCCTGGCAACTCAA
CAGAGCGCGCGAGTGAATGGCACACAGGCATGGCTGGCCAGAAAACTGATCA
GCGTATTGAAAAGCCAGTTGAAATCCTGGCTGTCTGAAACGGGTAATACGATC
GTCTTTAACCTGTGTAAAGCAGATGCTCAGCAAACATCGTTCGTTCGCACCGG
GCTTGCAGTGACAAAGCCTGAGACAACCAAAGGAGAGCGCCAGGGTGTAGGA
TCTCACGCCATCGATGCCTATTGTGCATTCTCAGTTGCAGCCGCTGAAGGCAT
GCACGAAACGCTCGCTCAAGGCGCGGGACTCATGGAGGATACCGAGTGGCTT
GCAACGGCGTTACCTGTTGACGTTGATGTGAATTGGGTTAAACGCCTACCTCTA
TATCGCAAGTCAGAACTAAGTACTCGGCCAATATTTAAAGAAGGAATTTATGCA
GAGTCATTTCTGACGACCTGGATTTCAGAGAAAGGAATTGAATCCGGCTTCTCA
TTGGGAAGCAATTCGGTGCAATGGCAAGGTAAGGATACTCGTGGGCTACTTGA
ATTACTGGCACCATTCGCCACTAAAAATTTAAATGTCGATACCGCCCTGGCAAG
CCCTCGCCCTGTACAGCTAGATATTAGTAAAGAAAAAGCGTTCAAGCACTTCGA
AATGGTGGCCAAAGCGCCGCATTCAGAGGTCGATCTGCATCAGGCGGATATTC
TTGATGCTCTCAGGTATTGCACCGCGAAAAAGGATATCAGTTCGCGTCTGATTG
ATGTACAGAAGAAAACACTGAAAAAACCAGAGGAAGTGCTAAAGGCTAAAGATA
TTTACGTGAAGGTTGATGTTGACGTTAAAGGCCTGGGTAAGTTAAAGGGCAAG
GTCACTCATCCGGCGATTGAGCAATGGAAAAGACTATTCCTTATCCCGGACATA
GAGACACGCATTGGGGAAAAAGCGGTAGGCATTAACTGGCAACAAATTGGTGA
AAAATACTTTGGTGAAGGATCGTCCCGCCCACACAGTAAAACGCGAAGGGTAT
ATTCGTTACCTATCGTCGATAGCCCTGACGGCGGCTATCGAATTAAAAGAATAG
CGCCTGATGGTTCAACGATCTGGCAACTGACGAGTATCAAAGATACGGGATCT
AGCGGGTTTACGGTAAATGACAAGGGTGTAAATTGGAAGCAACCAGTGGCTAT
CCCTGCGCTGCGTGATTCTTCGGTCGTTGGGGTTGGTGAACGTTACCAGCCTG
TTCCTGAGCAGCAAGTATACTTCGATGATTGGAAATGCGTAGACATAACGGATG
AGCGTGTGTTGTCAGTCTCAGTGGCTCCTGGTACTAAAGATCGGCGCTATGTC
TCAGTCAGTCAGACTCTATCTGATTTTAAGGCATGGTTAACCGAGAGTGGTATA

AAACTACATTCGCCTTTCGAAATTCCCTCAAGAGTCAAAGTGGATGCTGCTAAA
TTCCAGTCGGCACATGGCATTGACGTACTAGGAAAACCGAAATCGAATTTGTTT
ATTTCGAAGGTCGGGGATGTCGTTAACTATTGGTACATCAGAGAAAGTAGTCCT
GTTGTGATTAATCAGTATTACGACAAGGCACTCCTGGCAGAGCGCGAAGCAGT
TAACGAGACCGAGCCATGCGACATC

As the skilled person understand, if the polynucleotide of the invention has to be introduced in a mammal cell, specifically, in a human cell, the polynucleotide of the invention may comprise a nucleotide sequence encoding to the nuclease of the invention, wherein said nucleotide sequence has been humanized to be optimally expressed in the human cell. Thus, in another particular embodiment of the polynucleotide of the invention, alone or in combination with each and every one of the previous particular embodiments, the nucleotide sequence is humanized, preferably, the humanized sequence encoding the nuclease of the invention is the nucleotide sequence SEQ ID NO: 14.

SEQ ID NO: 14
ACCGACAGCAAGATCATCAGCCCTATCGCCATCGATCTTGGAGGTCTGT
ACACCGGCCTGTACATGGCCCAATACCCCTACGGCGAGAGCCTGGCCC
AGCACCACGGCAAGCTGACCACACTGGTGATGCCTGAGGAAGGCGGC
AAGATGACCTGGTCCCAGGCCGCCCGCACCGCCACACGCCACCGGAT
CCGGTCCAACAAGAGAAGAAAGCTGGCCAAGCGGCTGCTGAAGGTGAT
CTTCACGCAGGGCCTGAGCTACAAGCTGAACGACGCAGAACTTGAAGC
ACTGAACGGCCTGCTGAACCGTAGAGGCTACAATAGAGTGGAGACAGA
CATCGACCGGCAGCCCCTGGAGACCGTGCCTCCTAGCTGGATTGCAAA
TGAGTTCCCCGAGTTCTTCGACTCTAGCAGCCCCATTTCTGAACAACTG
GAAGAACTGCTCGAAGACATCCCTAAGCTGCGGACCTGGCCGCCGAC
GAGAAGTTCAATCTGAACAACAGAGAAGCCAAAAACAGCTGTTCACCG
ATGCTGAGGACGAGCAGGCTGACATCCTCAAGGCCTACGGATCCTGA
GAGAGAGCGTGACCGAGCTGCTGAATTCTATCGACTTCGGCCACCAGC
ACCGGAGAAAGTACCTGGAAGCCGTGGGCAAAGAGATCCTGAAAGACT
CGCGCCTGGCCGAGCTGCGGGCCAAGCTGGGCGCCTCCGCCCTGAAA
AACCTGATCGGAAACATCAGCAACCTGCAACTTCGGGCCCTGCGCTGG

TATTTTAATGATACCGCCATGAAGTCTGCTGATTACTACGACGGCGAAA
GATTCAAGTCTGTGTACGTGAGATGGCTGCAGAACTGGCACGTGGATG
AGGAAACTAGAGTCAACCGGAACAACGCCCTGAAGGTCATCCACCCCA
GCGAAGATATCCTGACAGCCCTGATGACGATCGACGCCGAGCTGACTA
TCCCGCCTTACGAGAACCAGAACAACCGCAGACCTCCGAAGGACCAGA
CCCTGTGGCTGAACCCCCAGAGTCTTAATGAGCAGTACGGCAGCCAGT
GGCGGGTGTGGGTGAATAATCTGAGGAAGAGAAACCCTGAGCTGGCCG
AGGGCATCGCCCCTGTGCTGGAATTCACCGACAGACGGTCCAGACTGA
AAACTAACAAGCAGCGGCTGACAGACACATCACTCTATGCGGATTCTTA
TTTCCTGCAGAGACTGTTGGACAGATCCAGAATTCACGACACATACGCC
CTGAGAGCAATTTCCAAGAACTTCAAGAGCGAGAAACTGGAAAAGTTCA
GATGGAACCTGAAACAGGACCTGGGCGACCAACACACCGAGAAGTTCC
TGGACTTCACCCAGAAGTATTACCAGGAGGTGGAGAGAGCCAAATCTG
GCATCTGGGAGCAGGAGAGTGCCAGCCTGCTGGAAAAGGCCGACATCA
ACCCTCCAAGTAAGCGGAAAATTCTGAACAGACTGGTCGGCAACATCCT
GGGAGTTGAGATGAGCGCCGAAGAGCTGACCACCTTCCTGTCGGACGT
ATGGACCAAGAAGGTGCACGGTCCTTCCACAGTGAAGAGCATCTGCAA
GGCCGCTGAGGATCTGCGGAAGCAGAACGGCAACCTGTTCAACATGTA
CATCCAGAGCGTGCGGTACAGAGCCTCTGTGGAAGCCGGCGCCGAGA
AGAAATTCAAGGGCTTAGATAAGGAAATCTGGCAGCTGCTGGTGAAGGT
AGAGAAAGCCGCCGACGTGATTAGCGGAAGTATCTCCGCCACAGAGAC
ACAAAAGCAGAGATTCAGCAACACCTTCTCTCTGGCGCAGCTGTACACA
CTGATCGAGACCGATAGAAACGGCTTCAGCAAGGTGTCTGCCGCTGCC
CATATGGAACTGGCCTGGAGAATGACAATGATCCCTGTGGATGACAAG
GAGGCCGCCAGATGCAGCAGACTGCCTGCCGATTCCAGCAGACCCTTC
GACGGCGTGCTGAGAAGACTACTGGAAAGACAGGCCTACGAGATCGCC
ACACTGAAGTCAGGCCAGATCAAGGAATCTGGAGTTCGGATTCTGAG
ATCAGCATCCCTGTGGTGGTGGAAGAAACCGCTTTACATTCAGCATCG
GCCTGGCCGACGTGAAGAAAGATGGAAGCAAACGCAAGAAACTGCTGT
CACTGGGCGAGAAACAGCTGGAAAAGTGGCAAGAGAAGAACGAGCGG
ATCCGGCAGGGAGGCTCTGGCCTGTGTCCTTATAAGGGCACCATCCTG
GACAAGGATGCCGAGCTGGACCACATCATCCCTCGGAGCTACAGCCGG

GACGCCAGCGGCACCATCTTTAACAGCGAAGCTAATCTGATCTACGCCA
GCAGACGAGGCAATCAGGAGAAAAGGACCAGAGATACGGCATCTCTG
ACCTGGCCCCTAACTACCTAAAGGCCGTGTACGGCACAAGCGACAGCG
CCGAAATTACCAGAATTATCGAGGCTAGCGTCGCCTCTATCAAGAAGGA
CTTCCTTTTAGCGAGCTCGAAGGCGATCAGCAGAGATGTCAGACAT
GCTCTGTTCCTGGAGAGAGACTCCAAGGCTTTGAGAAGGTGTTCCGG
AGACTGGCCACACAGCAGAGCGCCAGAGTGAATGGCACCCAGGCCTG
GCTGGCCAGAAAGCTGATCAGCGTGCTGAAGAGCCAACTGAAGAGTTG
GCTGAGCGAAACCGGCAACACCATCGTGTTTAATCTGTGCAAAGCTGAC
GCCCAGCAGACATCCTTCGTGAGAACTGGCCTGGCCGTGACCAAGCCC
GAGACAACCAAAGGAGAAAGACAGGGCGTGGGCTCCCACGCCATCGA
CGCTTACTGCGCCTTCAGCGTTGCGGCCGCCGAGGGCATGCACGAGA
CCCTGGCCCAGGGCGCCGGCCTGATGGAAGATACAGAATGGCTGGCC
ACCGCACTGCCTGTGGACGTGGACGTGAACTGGGTCAAGCGGTTACCT
CTCTACCGGAAGAGCGAGCTGAGCACCCGTCCAATCTTCAAGGAGGGC
ATCTACGCCGAGTCTTTTCTGACCACCTGGATCAGCGAAAAGGGCATCG
AAAGCGGATTTAGCCTGGGAAGCAACAGCGTGCAGTGGCAGGGCAAG
GACACCAGAGGCCTGCTGGAGCTCCTGGCTCCTTTCGCCACAAAAAAC
CTGAATGTGGACACCGCCCTGGCTAGTCCTAGACCCGTGCAGCTCGAT
ATCAGCAAAGAAAAGCTTTCAAACACTTCGAGATGGTGGCCAAGGCCC
CCCACTCTGAGGTGGATCTCCACCAGGCTGACATCCTGGACGCTCTGC
GGTACTGTACCGCTAAGAAGGACATTAGCTCTCGGCTGATAGATGTGCA
GAAGAAGACCCTGAAGAAGCCCGAGGAAGTGCTGAAGGCAAAGGATAT
ATACGTGAAGGTTGATGTGGACGTTAAGGGCCTGGGCAAGCTAAAGGG
CAAGGTGACACACCCTGCCATCGAGCAATGGAAACGACTGTTCCTGATT
CCAGATATCGAAACCAGAATCGGAGAGAAGGCCGTGGGCATCAACTGG
CAGCAAATCGGCGAAAAGTACTTTGGCGAGGATCTTCCCGGCCTCAC
AGCAAAACCAGAAGAGTGTATAGCCTGCCTATTGTGGATAGCCCTGACG
GCGGGTACAGAATCAAGAGAATCGCCCCAGATGGCAGCACCATCTGGC
AGCTGACCAGCATCAAGGACACCGGCAGCAGCGGCTTCACAGTGAACG
ACAAGGGCGTGAACTGGAAGCAGCCTGTGGCAATCCCTGCCCTGAGAG
ATAGCAGCGTGGTGGGCGTGGGAGAAAGATACCAGCCCGTCCCCGAG

CAGCAGGTGTACTTCGATGATTGGAAGTGCGTGGACATCACGGACGAG
CGGGTGCTATCCGTGTCCGTCGCCCCTGGCACAAAGGACAGAAGATAC
GTGTCCGTGTCCCAGACCTTAAGCGATTTCAAAGCTTGGCTGACCGAAA
GCGGAATCAAACTCCACAGCCCATTCGAAATCCCCAGCAGAGTGAAGG
TGGATGCTGCTAAGTTCCAGAGCGCCCATGGCATCGACGTGCTGGGAA
AGCCTAAGAGCAACCTCTTCATCTCTAAAGTGGGCGACGTCGTGAACTA
CTGGTACATCCGGGAGAGCTCTCCAGTTGTGATCAACCAATACTACGAC
AAGGCCCTGCTGGCTGAGCGCGAGGCTGTGAATGAGACCGAGCCTTG
CGACATC

### Vector of the invention

A polynucleotide comprising a nucleotide sequence encoding a nuclease of the invention, or the guide RNA (dual and/or single) of the invention, may be introduced into a cell.

Thus, in another aspect, the present invention relates to a vector, hereinafter "vector of the invention", comprising
- the polynucleotide of the invention, and/or
- the dual guide RNA of the invention; and/or
- the single guide RNA of the invention.

As used herein, the term "vector" or "plasmid" refers to any vehicle used to transfer foreign genetic material into a cell. Said vector can be a viral vector or a non-viral vector, such as a plasmid. By way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d, pTDTI, etc. Said viral and non-viral vectors can be administered to the cell by the conventional methods widely known in the state of the art.

In a particular embodiment of the vector of the invention, the vector is a plasmid, a retroviral vector, a lentiviral vector, an adenoviral vector, and an adeno-associated viral vector.

The vector may contain, among others, multiple cloning sites, expression regulating sequences, suitable replication origins for the host cell in which the vector is introduced, selection markers, etc. In a particular embodiment, the vector is an expression vector which allows the expression of the nucleotide sequence or polynucleotide in the target cell and generally has a promoter sequence (or expression regulating sequences) that drives expression thereof. The promoter sequence preceding the polynucleotide is operatively bound to the said polynucleotide. As used in this description, the expression "operatively bound" means that the polynucleotide is within the correct reading frame for their expression under the control of said regulating sequences. The regulating sequences useful for the present invention can be nuclear promoting sequences or, alternatively, enhancer sequences and/or other regulating sequences increasing the expression of the polynucleotide. The promoter can be constitutive or inducible. If the constant expression of the polynucleotide is desired, then a constitutive promoter is used. Examples of well-known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the polynucleotide is desired, then an inducible promoter must be used. In a non-induced state, the inducible promoter must be "silent". "Silent" is understood to mean that in the absence of an inducer, little or no expression of the polynucleotide is detected; in the presence of an inducer, however, the expression of the polynucleotide occurs. The expression level can frequently be controlled by varying the concentration of the inducer so that the inducible promoter is stimulated more strongly or weakly and consequently, the concentration of the polynucleotide transcribed product is affected. Examples of well-known inducible promoters are: an androgen or estrogen-responsive promoter, a doxycycline-responsive promoter, a metallothionein promoter, or a promoter responding to ecdysone. Other various examples are well known in the art and can be used for implementing the invention.

### Cell of the invention

In order to express the polynucleotide of the invention, the dual guide RNA of the invention; and/or the single guide RNA of the invention, they have to be introduced in an expression system such as a host cell. Taking this into account, the nucleotide sequence may be optimized for a proper expression in the chosen host cell. Thus, in another aspect, the present invention relates to a cell, hereinafter "cell of the invention", comprising (or consisting of):
- (i) the ribonucleoprotein of the invention, and/or
- (ii) the nuclease of the invention, and/or
- (iii) the polynucleotide of the invention, and/or
- (iv) the dual guide of the invention, and/or
- (v) the single guide of the invention, and/or
- (vi) the vector of the invention.

The term "host cell" means any cell type that is susceptible to transformation, transfection, transduction, and the like with the nucleotide sequences or vector of the invention.

The vector introduced into a host cell may be maintained as a chromosomal integrant or as a self-replicating extra-chromosomal vector. The term "host cell" also encompasses any progeny of a parent cell that is not identical to the parent cell due to mutations that occur during replication.

The host cell may be any cell, e.g., a prokaryote or a eukaryote such as a mammalian, insect, plant, or fungal cell. Any method known in the art for introducing DNA into a host cell can be used in the context of the present invention.

The prokaryotic host cell may be any Gram-positive bacterium or Gram negative bacterium. Gram positive bacteria include, but not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but not limited to, *E. coli, Pseudomonas, Salmonella, Campylobacter, Helicobacter, Flavobacterium, Fusobacterium, Neisseria,* and *Ureaplasma.* In a particular embodiment, the host cell is *E. coli.* Examples of *E. coli* strains useful for vector propagation and cloning procedures include, without limiting to, *E. coli* DH10B (Invitrogen), DH5α (ATCC, Invitrogene), DM1 (Invitrogen), GeneHogs (Invitrogen), HB101 (Bio-Rad, Invitrogene, Promega), INV110 (Invitrogen), JM109 (ATCC, Promega), JS5 (Bio-Rad), LE392 (ATCC), NM522 (AS), SCS110 (Stratagene), STBL4 (Invitrogen), SURE (ATCC, Stratagene), TG1 (Stratagene), XL10-Gold (Stratagene) and XL1-Blue MRF (Stratagene).

Examples of insect cells include, without limiting to, *Spodoptera frugiperda* cells (such as SF9 cell or SF21 cell or Sf900+), a BTI-Tn-5B1-4 insect cell from Trichoplusia ni (High-five^{™}, Invitrogen, Carlsbad Calif.), expresSF+^{®}, Drosophila Schneider 2 (S2) Cells, Se301, SeIZD2109, SeUCR1, MG-1, Tn368, HzAm1, Ha2302, Hz2E5 and High Five from Invitrogen.

Examples of the fungi host cells includes, without limiting to, Aspergillus (e.g. *Aspergillus awamori, Aspergillus fumigatus, Aspergillus foetidus, Aspergillus japonicus, Aspergillus nidulans, Aspergillus niger* or *Aspergillus oryzae* cell), Aureobasidium, Bjerkandera, Ceriporiopsis (e.g. *Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora),* Chrysosporium (e.g. *Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum*), Coprinus (e.g. *Coprinus cinereus*), Coriolus (e.g. *Coriolus hirsutus*), Cryptococcus, Filibasidium, Fusarium (e.g. *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell), Humicola (e.g. *Humicola insolens, Humicola lanuginosa*), Magnaporthe, Mucor (e.g. *Mucor miehei*), Myceliophthora (e.g. *Myceliophthora thermophila*), Neocallimastix, Neurospora (e.g. *Neurospora crassa*), Paecilomyces, Penicillium (e.g. *Penicillium purpurogenum*), Phanerochaete (e.g. *Phanerochaete chrysosporium*), Phlebia (e.g. *Phlebia radiata*), Piromyces, Pleurotus (e.g. *Pleurotus eryngii*), Schizophyllum, Talaromyces, Thermoascus, Thielavia (e.g. *Thielavia terrestris*), Tolypocladium, Trametes (e.g. *Trametes villosa or Trametes versicolor*), or Trichoderma (e.g. *Trichoderma harzianum, Trichoderma koningii, Trichoderma longibrachiatum, Trichoderma reesei, or Trichoderma viride* cell) cell.

The host cell may also be a yeast cell. "Yeast" as used herein includes ascosporogenous yeast (Endomycetales), basidiosporogenous yeast, and yeast belonging to the Fungi Imperfecti (Blastomycetes). More preferably, the yeast host cell is a Candida, Hansenula, Kluyveromyces (e.g. *Kluyveromyces lactis*), Pichia (e.g. *Pichia pastoris*), Saccharomyces (e.g. *Saccharomyces carlsbergensis, Saccharomyces cerevisiae, Saccharomyces diastaticus, Saccharomyces douglasii, Saccharomyces kluyveri, Saccharomyces norbensis, or Saccharomyces oviformis,* etc.), Schizosaccharomyces, or Yarrowia cell (*Yarrowia lipolytica*)*.*

The host cell may also be microalgae. Examples of microalgae include, without limiting to, microalgae from the genera *Chlamydomonas* (e.g. *Chlamydomonas reinhardtii*), *Botryococcus, Neochloris, Nannochloropsis, Phorphyridium, Scenedesmus, Chlorella, Tetraselmis* and *Spirulina.* The chloroplasts of these algae are particularly well suited to the production of bacterial proteins as it mimics the native bacterial expression environment of these proteins while being devoid of their cell wall target.

The host cell may also be a plant cell, wherein the vacuolar deposition of recombinant proteins in plant vegetative organs is used to increase yields. To achieve this, the nucleotide sequence of the invention may be fused to sequence specific vacuolar sorting signals (ssVSS) typical of proteases or proteinase inhibitors and/or Ct-VSS representative of storage proteins or plant lectins. Both types of motifs were capable to increase accumulation. Importantly, the type of VSSs or position, either the N or C-terminus, did not alter protein stability, levels or post-translational modifications. Examples of plant cells include, without limiting to, *Nicotiana* sp. (e.g. *N. tabacum, N. benthamiana,* etc.) sugarcane, tomato (*Solanum lycopersicum*) and carrot (*Daucus carota*)*.*

Examples of mammal cells which can be used in the context of the present invention include, without limiting to, a T-cell, a B-cell, an NK cell, epithelial cell lines (porcine, human, etc.), osteosarcoma cell lines (human, etc.), neuroblastoma cell lines (human, etc.), epithelial carcinomas (human, etc.), glial cells (murine, human, etc.), liver cell lines (monkey, etc.), CHO cells (ChineseHamsterOvary), COS cells, BHK cells, HeLa, 911, AT1080, A549, 293 or PER cells. C6, NTERA-2 human ECC cells, D3 cells of the mESC line, human embryonic stem cells such as HS293 and BGV01, SHEF1, SHEF2 and HS181, NIH3T3, 293T, REH and MCF-7 cells and hMSCs (human mesenchymal stem cells), and GliNS2 cells (glioma stem cells). In particular embodiment of the cell of the invention, the cell is mammal cell.

### Uses of the invention

The ribonucleoprotein complex, the dual and/or single guide RNA, and nuclease described herein are capable of recognizing, binding to, and optionally nicking, unwinding, or cleaving all or part of a target sequence.

Another aspect of the invention refers to the use of (i) the ribonucleoprotein complex of the invention, and/or (ii) the dual guide RNA of the invention, and/or (iii) the single guide RNA of the invention, and/or (iv) the vector of the invention, and/or (v) the nuclease of the invention, and/or (vi) the polynucleotide of the invention, for modifying *in vitro* target nucleic acid sequence, that comprises binding, cutting or cleaving specifically a target nucleic acid sequence, wherein the elements (i) to (vi) are not used for modifying the germ line genetic identity of human beings.

Another aspect of the invention refers to a ribonucleoprotein complex of the invention, and/or a dual guide of the invention, and/or a single guide of the invention, and/or a vector of the invention, and/or a nuclease of the invention, and/or a polynucleotide of the invention, for use in therapeutic modifying a target nucleic acid sequence that comprises binding, cutting or cleaving specifically a target nucleic acid sequence.

Alternative to the uses of the invention, the present invention also encompasses a method for modifying *in vitro* a target nucleic acid sequence, comprising putting into contact the ribonucleoprotein complex of the invention, and/or the dual guide RNA, and/or the single guide RNA of the invention, and/or the vector of the invention, and/or the nuclease of the invention, and/or the polynucleotide of the invention, with a target nucleic acid sequence, wherein the method is not a process for modifying the germ line genetic identity of human beings.

In addition, the present invention also encompasses a method for modifying a target nucleic acid sequence in a non-human eukaryotic cell, comprising contacting the non-human eukaryotic cell with the ribonucleoprotein of the invention, and/or a dual guide of the invention, and/or a single guide of the invention, and/or a vector of the invention, and/or a nuclease of the invention, and/or a polynucleotide of the invention.

In a particular embodiment of the use of the invention, cutting or cleavage of a target nucleic acid sequence is carried out in a temperature range of from 5°C to 45.5°C, preferably, from 5°C to 10°C, more preferably, at 5°C. In all ranges mentioned in the present description the extremes are included.

The concept to "modify in vitro a target nucleic acid sequence" refer herein to produce an altered target site. An "altered target site", "altered target sequence", "modified target site", "modified target sequence" are used interchangeably herein and refer to a target sequence as disclosed herein that comprises at least one alteration when compared to non-altered target sequence. Such "alterations" include, for example: (i) replacement of at least one nucleotide, (ii) a deletion of at least one nucleotide, (iii) an insertion of at least one nucleotide, (iv) a chemical alteration of at least one nucleotide, or (v) any combination of (i) - (iv). A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, (iv) a chemical alteration of at least one nucleotide, or (v) any combination of (i) - (iv).

All the definitions and particular embodiment of the uses of the invention are applicable to the method of the invention.

### DESCRIPTION OF THE DRAWINGS

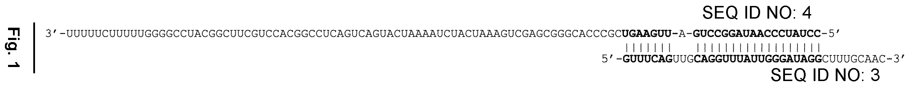

**Figure 1****.** Secondary structure of duplex guide RNA of the invention formed by pairing of two sequences, crRNA and tracrRNA, sense vs antisense. The antisense nucleotide sequence of tracrRNA is identified as SEQ ID NO: 4 and the sense nucleotide sequence of crRNA is identified as SEQ ID NO: 3.
**Figure 2****.** Sequence of single RNA guide of the invention.
**Figure 3****.** Dilutions of the IVT (in vitro translation) products were tested for cleavage activity on the PAM library by PCR amplification of the product containing the adapter ligated after the DNA cut. Legend: bands indicate activity in cutting plasmids. Controls:
   Ad, uncut library (negative control); Sp, PCR from protospacers (positive control).
**Figure 4****.** T7 endonuclease I assay to determine nuclease activity of nuclease of the invention and SpCas9 on human genes in Hek293T cell line.
**Figure 5****.** T7 endonuclease I assay to determine nuclease activity of nuclease of the invention and SpCas9 on human gene FANFC in K562 cell line. Digestion mixes containing 186 uM of protein and 350 uM of gRNA, except for DO2 Cas9 (b) where 248 uM of protein and 466 uM of gRNA were used.
**Figure 6****.** DO2 Cas9 activity in vitro at different temperatures using a PCR product (A) or a plasmid to be linearized (B) as DNA template. Linearized plasmid upon cut is visualized as a band lower than the uncut control. DO2 Cas9 (0,35uM) was incubated with gRNA (against dpy-10) for 30 minutes at 25°C to form Ribonucleoproteins (RNPs). RNPs were then frozen at -20°C for 2 hours. RNPs and DNA template were incubated for 5 minutes at the indicated temperatures, then mixed for DNA digestion for 30 minutes. Reaction was inactivated with bluejuice and 70°C for 10 min.
**Figure 7****.** (A) DO2 Cas9 and FnCas9 in vitro activities from 5°C to 50°C. (B) Semiquantitative score of activity. Both proteins were used at 0.35uM. RNP formation at 25°C for 30 minutes, and digestions at different temperatures for 30 minutes.
**Figure 8****.** Semiquantitative evaluation of in vitro activity for DO2 Cas9 (A) and FnCas9 (B) at different temperatures and three distinct protein concentrations.
**Figure 9****.** *In vitro* digestion of a PCR fragment containing the point mutation allele *ed3* at *unc-119* gene, using gRNA specific for such mutated target. 50 bp ladder was loaded in the first and last lines as a size marker.
**Figure 10****.** *In vitro* reaction at 37°C. DO2 Cas9 and SpCas9 cut efficiently when using gRNAs without mismatches but DO2 Cas9 activity drops when using gRNAs with mismatches.
**Figure 11****.** *In vitro* reaction at 20°C. DO2 Cas9 and SpCas9 cut efficiently when using gRNAs without mismatches. SpCas9 shows good activity even using gRNAs with mismatches at position +3 and +5 whereas DO2 Cas9 activity is more affected with mismatches at those positions.
**Figure 12****.** DO2 Cas9 (without gRNA) is thermosensitive compared to FnCas9. Challenging proteins at different temperatures for 1h. (A) Temperature range from 25°C to 40°C. (B) Temperature range from 25°C to 35°C. All reactions were frozen at -20°C for 1,5hrs. RNP formation at 25°C for 30 minutes. Digestion at 25°C for 30 minutes, inactivated with Bluejuice and heat (70°C for 10 min).
**Figure 13****.** gRNA provides thermostability to DO2 Cas9. Challenging ribonucleoproteins at different temperatures for 1h. RNP formation, challenge temperature, and digestion.
**Figure 14****.** Temperature dependence of circular dichroism (CD) spectra (ellipticity) at 222 nm for (A) DO2 Cas9, (B) FnCas9 (from SIGMA) and (C) SpCas9.
**Figure 15****.** Temperature dependence of fluorescence intensity at 330 nm for (A) DO2 Cas9, (B) FnCas9 (from SIGMA) and (C) SpCas9.
**Figure 16****.** Scheme of DO2 Cas9 genome editing in *C*. *elegans.* Scoring in F1 or F2 generation. 27 worms were edited with DO2 Cas9 among 45 well injected animals (Green fluorescence as marker of a good microinjection), which implies 60% of efficiency for DO2 Cas9 in genome editing in animals.
**Figure 17****.** Sequences of *C*. *elegans dpy-10* region targeted *in vivo* with DO2 Cas9 RNPs showing insertions and deletions compatible with the predicted cut site.
**Figure 18****.** Zebrafish embryos were microinjected with DO2 Cas9 mRNA (225pg) and gRNA (150pg), at two temperatures (25°C and 33°C) with different gRNAs against albino gene (g1, g2, and g3) and a control gRNA. (A) percentage of embryos showing the expected phenotype. (B) Scored albino phenotypes.
**Figure 19****.** Editing of the slc45a2_g zebrafish gene using mRNA or purified protein to provide DO2 Cas9.
**Figure 20****.** Mutagenesis efficiency of DO2 Cas9 in N. benhtamiana assayed by transient expression. Mutagenesis efficiency (%) of DO2 Cas9 in four different N.
benthamiana genes. Two different guide RNAs were employed, one targeting two PDS genes and a second one targeting two SU genes. The mutagenesis efficiency was calculated using ICE Analysis (Synthego).
**Figure 21****.** (A) Structural similarities between the two Class II type II-B Cas9 proteins FnCas9 and DO2 Cas9. (B) AlphaFold model of the PAM recognition domain of DO2 Cas9.

### EXAMPLES

Next, the invention will be illustrated through tests carried out by the inventors that reveal the capacity of the nuclease of the invention to edit DNA sequences.

### Example 1. Sample collection and identification of nuclease

### 1.1 MATERIALS AND METHODS

### 1.1.1 Sample collection and generation of the Malaspina Vertical Profiles Gene Database

Cas9 sequence derives from the sequencing of metagenomes from the Malaspina Expedition 2010, a circumnavigation of the research vessel Hesperides that collected samples in from 33 sea stations, including 13 vertical profiles with 7 depths from surface to 4000 m. Specifically, Cas9 was identified in a sample collected at 2500 meters deep in the Atlantic Ocean (station 141). Extracted DNA was sequenced on the Illumina HiSeq 2000 platform, in a paired-end mode (2×101bp). Metagenomicfrom Fastq files analysis was carried out with the following pipeline: sequencing reads were assembled with MegaHit v1.1.3, prokaryotic contigs (contiguous fragments of DNA sequence from an incomplete draft genome) were filtered with Tiara v1.0.2, and genes were predicted with Prodigal in Prokka v1.15 on the assembled contigs. Genes were clustered at 95% of sequence similarity with CD-HIT-est v4.6.1 to produce a 17.4 million non-redundant gene catalog and 2,672 metagenome-assembled genomes (MAGs) of Archaea and Bacteria (Malaspina-VP-MAGs).

### 1.1.2 Identification of nucleas

The 17.4 million non-redundant gene catalog metagenomes of the Malaspina Expedition were examined, identifying 528,589 genes that shared any similarity to any of the 437 Cas profiles that formed our custom database. Out of these, only 4,707 genes were considered to belong to a CRISPR loci. In total, 1,146 CRISPR loci were detected from which 70% were classified unambiguously based on the majority rule output, and 43% contained at least one effector protein and therefore, were considered complete loci. All Cas9 proteins detected were retrieved in the 1,146 CRISPR-Cas loci predicted in the Malaspina Gene Catalog. After discarding truncated proteins, a collection of 48 Cas9 proteins was obtained. Seven type-II Cas9 protein sequences were chosen for further validation based in their low homology with other Cas9 proteins, the diverse subtypes (II-A, II-B or II-C), and their suitability to identify the trRNAs.

A phylogenetic tree of Cas9 protein was computed with RAxML v8.2.12 with PROTGAMMALG as the substitution model and 100 bootstrap samplings. The tree included sequences and information about all Cas9 proteins found in the detected loci. The pipeline used was based on Fonfara et al., 2014 (Fonfara et al., Nucleic Acids Res. 2014 Feb;42(4):2577-90). A set of nine Cas9 proteins was used as queries to perform a Position-Specific Iterated (PSI)-BLAST against the UniRef90 database.

### 1.2 RESULTS

The phylogenetic tree classified nuclease of invention (also called hereinafter as DO2 Cas9), as a Class II Type II-B Cas9 of 1,416 amino acids (SEQ ID NO: 1), which was isolated from uncultured bacteria in the deep ocean.

The nuclease of the invention is smaller (1,416 amino acids) than canonical FnCas9 (1,629 amino acids) (Class II Type II-B), which was isolated from *Francisella Novicida.* In addition, the nuclease of the invention presents 22.9% of identity with canonical FnCas9.

### Example 2. Identification of crRNA and tracrRNA to form the gRNA

### 2.1 MATERIALS AND METHODS

A total of seven CRISPR-Cas loci were analyzed by:
i) Predicting the CRISPR array and Cas genes with pilerCR (v1.06), CRISPRCasFinder (v1.1.2) and prokka (v1.14.6).
ii) Aligning the CRISPR DNA (CR) to the locus contig with blast (v2.7.1; -blastn-short -dust no) and considering alignments within 500 bp upstream or downstream of the CRISPR-Cas locus as putative antirepeats (AR) for further analyses.
iii) Looking for Rho-independent terminators (RTS, Rho-independent-like Termination Signal) that are close to the putative antirepeats (FindTerm webserver).
iv) Looking for promoters in the vicinity of the putative antirepeat with BPROM webserver.
v) Checking the interaction between the antirepeat and the CRISPR DNA in IntaRNA (v3.3.1).

### 2.2 RESULTS

When all conditions were checked, the sequences read from 5' to 3' from the antirepeat to the RTS with the poly-U tail are given as putative tracrRNA sequence for each locus. The transcription sense of the CRISPR repeats was inferred from the best CR-AR interactions, assuming this synteny of the tracrRNA. All coordinates are referred to the original contig coordinates in the plus strand.

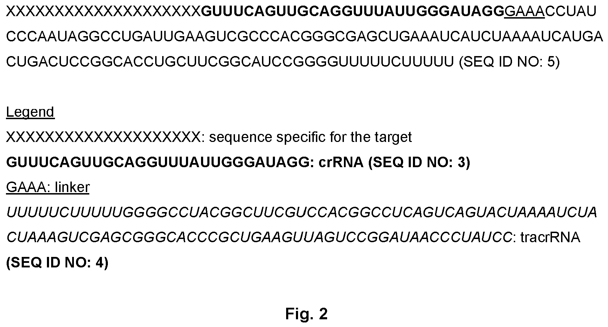

Such RNA duplex can work as gRNA (dgRNA) by adding at the 5' end 20 nucleotides corresponding to the 20 nucleotides upstream of the PAM at targeted sequence, or can be shortened to form the following guide RNA (gRNA) of 156 base pairs, which was found functional, by adding a 4 nucleotide GAAA linking a shorter crRNA to shorter tracrRNA to make this final gRNA sequence (sgRNA), being the first 20nt target-specific (Figure 2). Thus, sequences for tracrRNA, crRNA and gRNAs are defined in Table 1.

### Table 1. List of oligonucleotides discover in this study. All the sequences are presented, as conventionally, in 5'-3' sense, with the exception of the first sequence corresponding to DO2 Cas9 tracrRNA that is presented in 3'-5' sense (antisense).

| **Name** | **Sequence** | **Base** |
|---|---|---|
| DO2 Cas9 tracrRNA | SEQ ID NO: 4, Figure 1 (antisense) | RNA |
| DO2 Cas9 crRNA | SEQ ID NO:3, Figure 1 (sense) | RNA |
| DO2 Cas9 dgRNA | Pairing of these two sequences, sense (DO2 Cas9 crRNA) vs antisense (DO2 Cas9 tracrRNA), gives the RNA structure of the Figure 1 | RNA |
| DO2 Cas9 sgRNA | SEQ ID NO: 5, Figure 2 | RNA |

### Example 3. In vitro translation and transcription, and ribonucleoprotein (RNP) activity on plasmids dependent on the PAM sequence.

### 3.1 MATERIALS AND METHODS

### 3.1.1 In vitro translation and transcription, and RNP activity on plasmids.

Nuclease of the invention was produced by in vitro translation (lVT) using an *E. coli* cell-free transcription-translation system. gRNA was synthetized by T7 in vitro

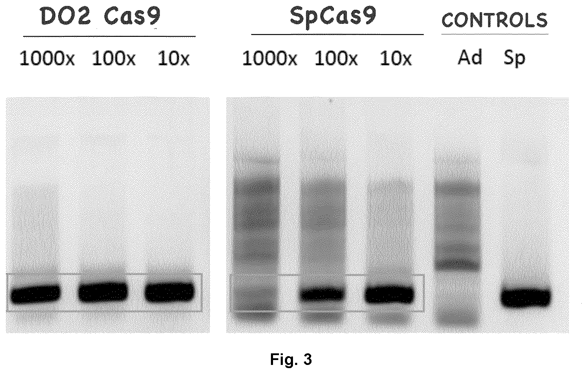

Transcription. The nuclease of the invention was assembled with their corresponding sgRNAs to form nuclease-gRNA complexes (Ribonucleoproteins (RNPs)) that were incubated with a library of plasmids with randomized PAMs sequences of 7 nucleotides. Then, a reaction to ligate an adapter was performed and cleaved products with the adapter incorporated were amplified by PCR and sequenced to determine the PAM sequence. Three different dilutions of the IVT (*in vitro* translation) product were tested for cleavage activity of the PAM library by PCR amplification of the product containing the adapter ligates after the DNA cut (Figure 3).

### 3.1.2 Identification of the PAM sequence

To identify the PAM preferences of Cas9 in vitro, a PAM plasmid library was cleaved with RNPs, adapters were ligated to cleaved fragments and Illumina libraries enriched in cleaved products were PCR amplified and sequenced (Karvelis et al. A pipeline for characterization of novel Cas9 orthologs. Methods Enzymol. 2019;616:219-240)*.*

In brief, 1µg of plasmid library in 90µL of reaction (R) buffer was prepared for each RNP concentration to be tested. Using RNPs assembled directly from E. coli lysate or IVT reactions, series of dilutions or distinct concentrations were prepared (e.g., 10X, 100X, 1000X) in CA buffer (10mMTris-HCl, pH 7.5 at 37°C, 100mM NaCl, and 1mM DTT). Next, cleavage reactions are initiated by adding 10ul of the Cas9 RNP complex into the reaction tubes containing 1µg of plasmid library and R buffer (total reaction volume 100µL). Reactions are gently mixed and allowed to proceed for 1h at 37°C. The samples after cleavage were stored at 20°C.

### 3.2 RESULTS

Upon in vitro translation (NT), DO2 Cas9 showed high cleavage activity on plasmids with specific PAM sequences (Figure 3).

Junctions generated by nuclease of the invention cleavage and adapter ligation at different positions of the protospacer (targeted region) were analyzed. PAM preferences at different dilutions were then determined as described in Karvelis et al. 2015 (*Karvelis et al. Rapid characterization* of *CRISPR-Cas9 protospacer adjacent motif sequence elements. Genome Biol. 2015 Nov 19;16:253*), and represented as Position Frequency Matrices (PFMs) (Table 2).

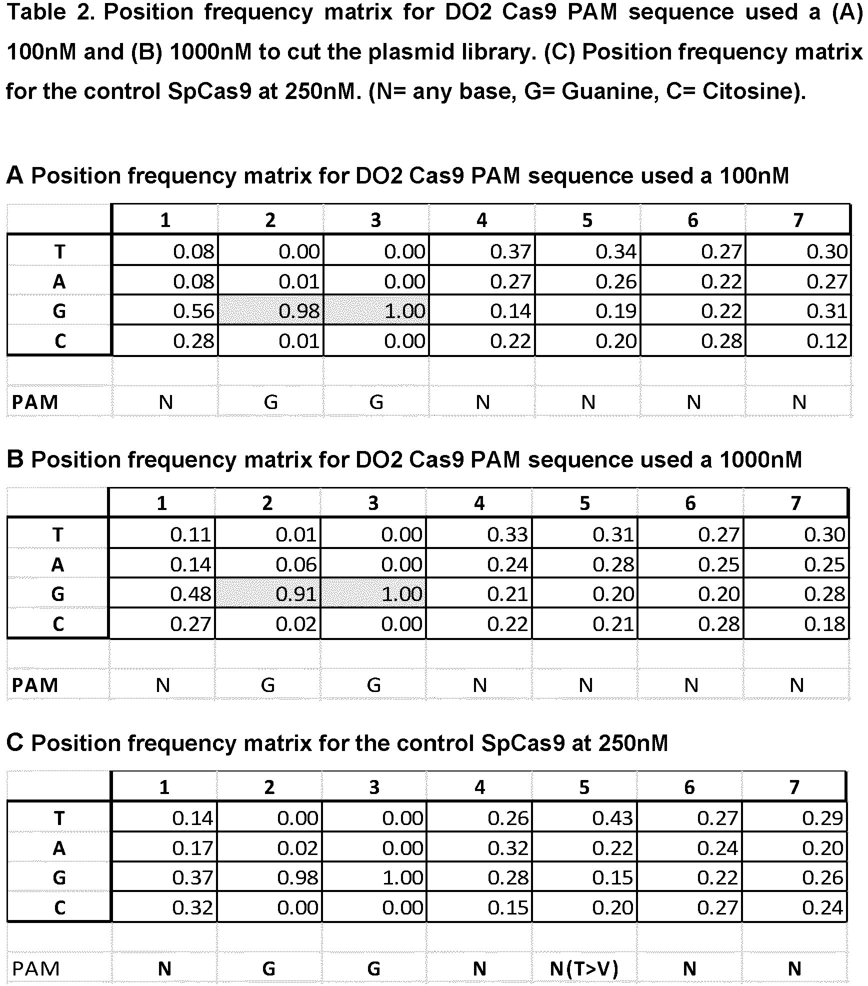

Determined Cas9 PAM was: NGG wherein N is any base, G is Guanine. N at position 1 of the 7 nucleotides PAM is preferentially G at lower concentration, and S (G over C where C is Citosine) at higher concentration. Guanines at position 2 and 3 are essential requirements.

In target strand, most of the cleavage events (>95%) occurs in the 3nt behind the space but in the non-target strands cleavage occurs predominantly at position -5 and -6. Consequently, DO2 cleavage leaves mostly overhangs of 2 or 3 nucleotides (Table 3).

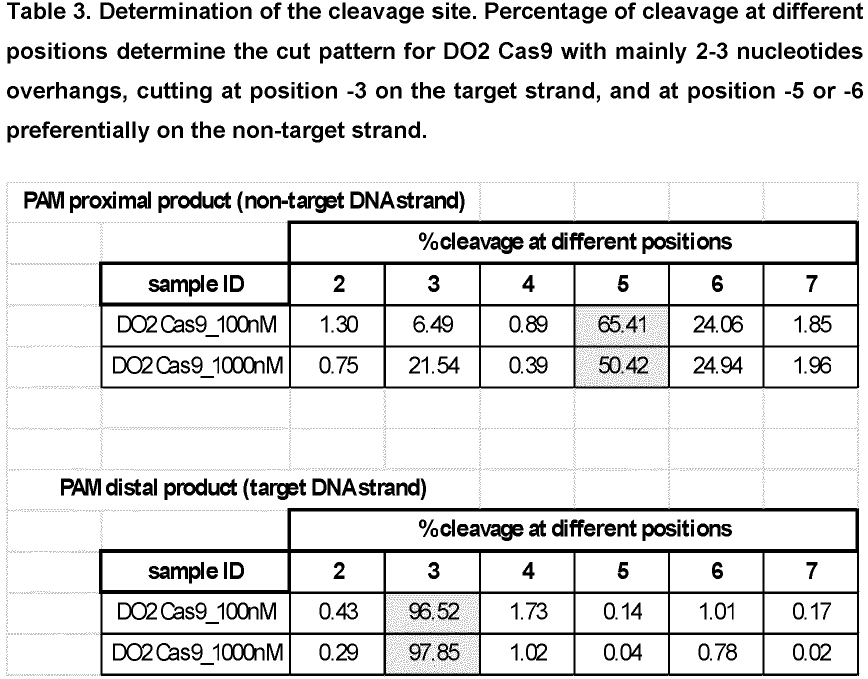

### Example 4. Evaluation of nuclease activity in human cell lines.

### 4.1 MATERIALS AND METHODS

### Use of DO2 Cas9 as plasmid

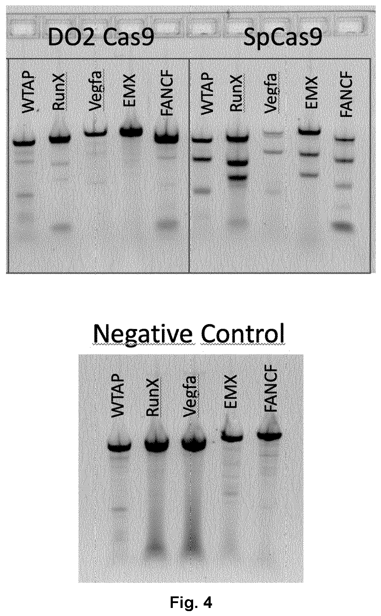

Sequence of DO2 Cas9 was human codon optimized using genscript algorithms. A SV40 Nuclear Location Signal (NLS) (CCGAAAAAGAAGCGAAAGGTC (SEQ ID NO: 12) was added at the N-terminus and a Nucleoplasmin NLS at C-terminus (AAAAGGCCGGCGGCCACGAAAAAGGCCGGCCAGGCAAAAAAGAAAAAG (SEQ ID NO: 60)). The resulting sequence (SEQ ID NO: 13) was cloned into the pcDNA3.1(+)-P2A-eGFP vector over Kpnl and Xbal sites. Kpnl [GGTACC], Xbal [TCTAGA] restriction sites were avoided. eGFP is cleaved from the protein by P2A enzyme and allows monitoring the expression level of Cas9. Plasmid and sgRNA geneblocks were transfected into a Hek293T cell line using the FuGene transfection agent (Reagent and DNA ratio 3:1). Cleavage activity in human genes FANCF1m, FANCF2m, EMX, Vegfa1 and Vegfa2 was analyzed 4 days incubation period after transfection (Figure 4).

### Use of DO2 Cas9 as RNPs

Purified DO2 Cas9 (see next section for information on purification) was also used to edit human cells (K562 cell line). RNP mixes for DO2 Cas9, FnCas9 and SpCas9 RNPs (ca. 186 uM protein), and 350 uM gRNA for a target in the FANFC gene, were incubated 10 minutes at 25°C and left on ice for 10 minutes before being added to the cells. A higher concentration of DO2 Cas9 was tested (248 uM protein and 466 uM gRNA). Transfection was performed with the Neon device, using a Neon tip with 100uL of the cells + Buffer R + RNP mix, using the program: K562 - 1450v, 10ms, 3 pulses.

In the Table 4, the specific spacers for the gRNAs used for targeting each gene in Figure 4 are shown.

### Table 4. Specific spacers (DNA targeted region) for the gRNAs used for targeting each gene are shown.

| **Name** | **Sequence** | **Base** |
|---|---|---|
| FANCF | TCCAAGGTGAAAGCGGAAGT (SEQ ID NO: 6) | DNA |
| RUNX | GAAGAGGGTGCATTTTCAGG (SEQ ID NO: 7) | DNA |
| EMX | GCTTCGTGGCAATGCGCCAC (SEQ ID NO: 8) | DNA |
| VEGFA | GACCCCCTCCACCCCGCCTC (SEQ ID NO: 9) | DNA |
| WATP | GGGAAAGGTAATCGAACTGT (SEQ ID NO: 10) | DNA |

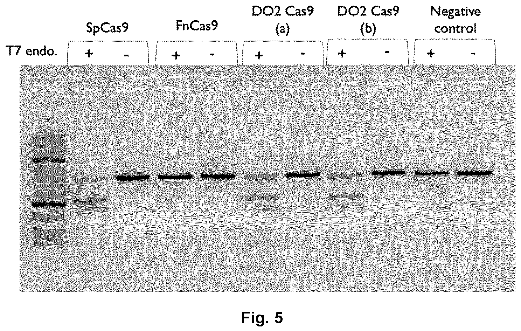

In Table 5, the FANFC spacers for the gRNAs used in Figure 5 for distinct Cas9 nucleases are shown.

### Table 5. FANFC spacers (DNA targeted region) for gRNAs used for the different Cas9 nucleases.

| **Name** | **Sequence** | **Base** |
|---|---|---|
| SpCas9 | GGAATCCCTTCTGCAGCACC (SEQ ID NO: 11) | DNA |
| FnCas9 | GGAATCCCTTCTGCAGCACC (SEQ ID NO: 11) | DNA |
| DO2 Cas9 | GGAATCCCTTCTGCAGCACC (SEQ ID NO: 11) | DNA |

gRNAs were synthetized by using HiScribe^{®}; Quick T7 High Yield RNA Synthesis Kit (NEB #E2050) from gblocks with T7 promoters.

SEQ ID NO: 13. Humanized D02 Cas9 with nuclear location signals:

CCGAAAAAGAAGCGAAAGGTCACCGACTCAAAAATCATCAGCCCTATTGCCAT
CGATCTCGGCGGCCTGTACACCGGTCTCTACATGGCCCAGTACCCTTACGGCG
AAAGCCTGGCCCAGCACCACGGCAAGCTCACAACACTGGTGATGCCTGAAGA
AGGCGGCAAGATGACCTGGAGCCAGGCCGCTAGAACAGCCACCCGGCACAG
GATCAGAAGCAACAAGCGGAGAAAGCTGGCCAAGAGACTGCTGAAGGTGATC
TTCACCCAGGGCCTGAGCTACAAGCTGAATGACGCCGAGCTGGAGGCCCTGA
ATGGCCTGCTGAACAGGAGGGGCTACAACAGAGTTGAGACCGACATCGATCG
GCAGCCTCTGGAGACCGTGCCCCCTTCTTGGATCGCCAATGAGTTCCCCGAAT
TCTTCGACAGCTCTAGCCCTATCAGCGAGCAGCTGGAGGAACTGCTCGAGGAC
ATCCCCAAGTTAAGGGACCTGGCTGCTGATGAAAAGTTCAATCTGAACAACCG
GGAAGCCAAGAAACAGCTGTTTACAGACGCCGAGGATGAGCAGGCAGATATC
CTGAAGGCTTATGGCATCCTGCGGGAATCTGTGACCGAGCTGTTAAACAGCAT
CGACTTTGGACACCAGCACAGACGGAAATACCTGGAGGCCGTGGGCAAGGAG
ATCCTGAAAGATAGCAGACTGGCCGAACTTAGAGCCAAACTGGGCGCCAGCG
CCCTGAAGAACCTGATAGGAAACATCAGCAACCTGCAGCTACGGGCCCTGAG
GTGGTATTTTAATGATACCGCCATGAAGAGTGCTGACTACTACGACGGCGAAA
GATTCAAGAGCGTGTACGTGAGATGGCTGCAAAACTGGCACGTAGACGAGGAA
ACGCGGGTGAACAGGAACAACGCCCTGAAGGTGATCCACCCAAGCGAGGACA
TCCTGACAGCTCTGATGACCATCGACGCCGAGCTGACTATCCCTCCTTACGAG
AACCAGAACAACCGGAGACCACCTAAGGACCAGACCCTGTGGCTGAATCCCCA
GAGCCTGAATGAACAATACGGCTCTCAGTGGCGGGTGTGGGTCAACAACCTGA
GAAAACGGAACCCCGAGCTGGCCGAAGGCATCGCCCCTGTGCTGGAGTTCAC
AGACAGAAGAAGCAGGCTTAAGACAAACAAGCAGAGACTGACAGACACCTCTC
TGTACGCTGACAGCTACTTCCTGCAGCGGCTCCTCGACCGGAGCCGCATTCAC
GACACCTACGCCCTCAGAGCAATCTCCAAAAACTTCAAGAGTGAAAAGCTGGA
GAAATTCCGGTGGAACCTGAAGCAGGACCTGGGCGACCAGCATACCGAGAAG
TTCCTGGATTTCACCCAAAAGTACTATCAGGAGGTGGAAAGAGCTAAGTCTGG
CATCTGGGAGCAGGAATCTGCGTCTCTCCTGGAAAAGGCGGATATCAACCCTC
CAAGCAAGCGGAAGATCCTCAATCGCCTGGTCGGAAATATCCTGGGCGTGGAA

ATGAGCGCCGAAGAGCTGACCACCTTCCTGTCTGATGTGTGGACCAAAAAGGT
GCACGGCCCTAGCACCGTGAAGAGCATCTGCAAGGCCGCCGAGGACCTGCGC
AAGCAAAACGGCAACCTGTTCAACATGTACATCCAGAGCGTGAGATACCGGGC
CAGTGTGGAAGCTGGCGCTGAGAAGAAATTCAAAGGCCTGGACAAAGAGATCT
GGCAACTGCTGGTGAAAGTTGAGAAGGCCGCCGATGTGATCAGCGGAAGCAT
CAGCGCCACAGAAACCCAGAAGCAGAGATTCAGCAATACCTTCAGCCTGGCCC
AGCTGTACACCCTGATTGAGACGGACAGAAACGGCTTCAGCAAGGTGTCCGCC
GCGGCCCACATGGAACTGGCCTGGAGAATGACAATGATCCCCGTGGATGACA
AAGAGGCCGCCCGGTGCAGCAGGTTGCCTGCTGATTCTAGCAGGCCCTTTGAT
GGCGTGCTGCGCAGACTGCTGGAGCGGCAAGCCTACGAGATCGCCACACTGA
AGTCCGGCCAGATCAAGGAAAGCGGCGTGAGAGACAGCGAGATCAGCATCCC
CGTGGTCGTCGAGGAAAACCGGTTTACCTTTAGCATCGGACTGGCTGATGTGA
AAAAAGATGGCTCCAAAAGAAAGAAGCTGCTGTCTCTGGGAGAAAAGCAGCTG
GAGAAGTGGCAGGAGAAGAACGAGAGAATTAGACAGGGAGGCAGCGGACTGT
GTCCTTACAAGGGCACCATCCTGGACAAGGATGCCGAGCTGGACCACATCATC
CCCCGGAGCTACAGCAGAGACGCCAGCGGCACAATCTTCAACTCTGAGGCCA
ACCTGATCTACGCCAGCCGGAGAGGCAATCAGGAGAAGAAGGACCAACGGTA
CGGCATCTCCGATCTGGCCCCTAACTACCTGAAGGCCGTGTACGGAACCAGC
GATAGCGCCGAGATCACCAGAATTATCGAGGCCTCCGTGGCTTCTATCAAGAA
AGACTTCCTGTTCAGCGAGCTTGAAGGCGACCAGCAACGGGATGTGCGACATG
CCCTGTTTCTGGAAAGAGATAGCAAGGCTTTCGAGAAAGTGTTCAGACGTCTTG
CTACACAGCAGAGCGCCAGAGTGAATGGCACCCAGGCTTGGCTCGCCAGAAA
GCTGATCTCCGTGCTGAAGAGCCAGCTGAAGTCCTGGCTGTCTGAAACAGGCA
ACACCATCGTGTTCAACCTGTGCAAGGCCGATGCTCAACAGACATCATTCGTC
CGGACCGGCCTGGCTGTGACCAAGCCCGAGACAACCAAGGGCGAGAGACAG
GGCGTGGGCAGCCACGCCATCGACGCCTACTGCGCCTTCTCGGTCGCCGCCG
CCGAGGGCATGCACGAGACCCTGGCCCAGGGGGCCGGCCTGATGGAAGATA
CCGAGTGGCTTGCTACAGCCCTGCCTGTGGACGTGGATGTGAACTGGGTGAA
GAGACTGCCTCTGTACAGAAAATCTGAATTGAGCACCCGGCCCATCTTCAAGG
AAGGCATCTATGCTGAATCTTTTCTGACAACCTGGATCTCTGAGAAGGGCATCG
AGAGCGGATTCAGCCTCGGCAGCAATTCTGTGCAGTGGCAGGGCAAGGATAC
CCGGGGTCTTCTGGAACTGCTGGCCCCTTTTGCCACCAAGAACCTGAACGTGG
ACACCGCCCTGGCCTCCCCTAGACCCGTGCAGCTGGACATCAGCAAGGAGAA
GGCCTTTAAGCACTTCGAGATGGTGGCCAAGGCCCCGCACTCTGAAGTGGAC

CTGCACCAGGCCGACATCCTGGACGCCCTGAGATATTGTACAGCCAAGAAGGA
CATCTCCAGCAGACTGATCGACGTCCAGAAGACCCTAAAGAAACCTGAAG
AGGTGCTGAAGGCCAAGGACATCTACGTGAAAGTGGACGTGGACGTAAAGGG
CCTGGGGAAACTGAAAGGCAAGGTGACCCACCCCGCTATCGAGCAGTGGAAG
AGACTGTTCCTGATCCCAGATATTGAGACAAGAATCGGCGAGAAGGCCGTGGG
CATCAACTGGCAGCAGATCGGCGAGAAGTACTTCGGCGAAGGCTCCTCTCGG
CCCCATAGCAAGACCAGAAGGGTTTACAGCCTGCCAATCGTGGACTCTCCTGA
TGGAGGATACCGGATCAAAAGAATCGCCCCTGACGGCTCAACCATCTGGCAGC
TGACAAGCATCAAGGACACAGGATCCAGCGGTTTCACCGTGAACGACAAGGG
CGTGAACTGGAAGCAGCCTGTTGCCATCCCTGCCCTGAGAGATAGCAGCGTG
GTGGGCGTGGGCGAGAGATACCAGCCAGTGCCTGAGCAGCAGGTGTACTTCG
ACGACTGGAAGTGCGTGGACATTACAGACGAGAGAGTGCTGTCCGTGAGCGT
GGCCCCTGGTACAAAGGACAGACGGTACGTGAGCGTTAGCCAGACCCTGAGC
GATTTCAAGGCATGGCTGACCGAGAGCGGGATCAAGCTGCACAGCCCCTTCG
AGATCCCAAGCCGGGTCAAAGTGGACGCCGCTAAGTTTCAGAGCGCCCATGG
CATCGACGTGCTGGGCAAGCCTAAGAGCAACCTGTTCATCTCTAAGGTCGGAG
ATGTGGTGAACTACTGGTACATCAGAGAATCCTCCCCTGTGGTCATAAACCAGT
ACTACGACAAGGCTCTGCTGGCCGAAAGAGAGGCTGTGAACGAGACCGAGCC
CTGCGACATCAAAAGGCCGGCGGCCACGAAAAAGGCCGGCCAGGCAAAAAAG
AAAAAG

### 4.2 RESULTS

### Use of Cas9 as plasmid

DO2 Cas9 activity was observed in human Hek293T (Figure 4).

### Use of Cas9 as RNPs

As a result of T7 assays, editing efficiency for DO2 Cas9 comparable to that of SpCas9 was observed (Figure 5). No significant improvements were detected with increasing DO2 Cas9 protein concentration. FnCas9 activity was very low.

### EXAMPLE 5. In vitro nuclease activity

### 5.1 MATERIALS AND METHODS

### Protein purification

DO2 Cas9 sequence, *E. coli* optimized, plus the Nuclear Location Signal (NLS) SV40 (SEQ ID NO: 14), and the 6xHis tag (SEQ ID NO: 15), was cloned into the vector pET-21(+) (pET21_DO2 Cas9) for expression in *E. coli* (Rosseta) induced with IPTG (0.5 mM final concentration). Culture pellet was washed with TrisHCl 50 mM pH 8 and kept at -80°C. Cell pellet (buffer: 50 mM HEPES KOH pH 7.5, 500 mM KCI, 10 glycerol, 10 mM Imidazol) was lysated by sonication at 20% (cycles 2 seconds on, 10 seconds for five minutes). After centrifugation (30 min at 10.000g), and filtering (0.45 µm filter) sample was loaded in a chromatography column *His Trap FF.* Elution buffer was 50 mM HEPES KOH pH 7.5, 500 mM KCI, 10 glycerol, 500 mM Imidazol. Fractions with enrichment in DO2 Cas9 were collected and loaded in a molecular exclusion chromatography column *HiPrep 16*/*60 Sephacryl S200HR* pre-equilibrated with the buffer containing 50 ml HEPES KOH pH 7.5, 250 mM KCI, and 10% glycerol. Purified protein was kept in aliquots in a buffer solution containing 25 mM HEPES·KOH, 125 mM KCI, 40% v/v glycerol, and 1 mM DTT.
SEQ ID NO: 14 Sequence of 21 nucleotides coding for the 7 amino acids of the SV40 NLS.
   CCGAAGAAGAAACGAAAAGTT
SEQ ID NO: 15. DO2 Cas9 codon optimized for *E. coli* expression plus the NLS SV40 and the 6xHis tag.

ATGAAGATTGAGGAGACGGATAGTAAGATAATTAGTCCTATAGCCATAGATCTT
GGTGGACTCTACACAGGCCTTTACATGGCACAGTACCCTTACGGTGAAAGTCT
GGCACAACACCACGGTAAATTGACGACTTTGGTCATGCCGGAAGAAGGCGGAA
AGATGACTTGGAGCCAGGCAGCCCGCACGGCCACACGCCATAGAATTCGCTC
CAATAAGCGCCGCAAACTCGCTAAGCGCCTCTTGAAGGTTATTTTCACACAGG
GCTTAAGCTATAAACTCAACGACGCCGAGTTAGAAGCTCTGAACGGGCTGCTT
AACAGAAGAGGTTATAATCGGGTTGAGACAGACATCGACCGACAGCCCCTGGA
AACGGTGCCCCCAAGCTGGATCGCAAACGAATTTCCTGAGTTCTTTGACTCCTC
TTCACCGATCAGTGAACAGTTAGAGGAGTTATTAGAGGATATCCCGAAGCTCC
GCGACCTGGCCGCGGACGAGAAATTCAACCTGAACAACCGGGAAGCGAAGAA

GCAACTTTTCACCGATGCTGAGGATGAGCAGGCGGACATACTCAAGGCATATG
GCATCCTGCGTGAGTCCGTGACCGAACTCCTGAATAGCATTGATTTTGGTCATC
AACACCGGCGTAAGTATTTAGAGGCGGTGGGCAAGGAAATCCTGAAAGACTCT
CGTCTGGCCGAATTACGCGCAAAACTGGGCGCCAGCGCTCTTAAGAACCTGAT
CGGGAATATAAGTAATCTTCAACTCCGTGCATTGCGGTGGTATTTCAATGACAC
GGCCATGAAATCTGCAGACTATTACGACGGGGAGAGATTTAAATCTGTCTACGT
GCGCTGGCTCCAAAATTGGCACGTAGATGAGGAGACTCGTGTCAACAGAAATA
ACGCGCTTAAGGTCATCCACCCAAGTGAGGACATACTGACAGCTCTGATGACT
ATCGACGCGGAGCTCACTATCCCGCCTTACGAGAACCAAAACAATCGCCGCCC
GCCGAAGGACCAAACTCTGTGGTTGAATCCGCAATCTTTGAACGAACAGTACG
GGTCGCAATGGCGCGTGTGGGTCAACAACCTGCGAAAGAGAAACCCTGAATTA
GCTGAAGGCATTGCCCCGGTATTGGAATTTACAGATAGACGCTCTCGGCTGAA
GACGAACAAACAACGTTTAACGGACACTTCACTTTACGCCGATTCCTACTTCCT
GCAACGCTTGCTGGACAGAAGTCGTATTCACGACACGTATGCGCTTCGCGCTA
TTTCGAAGAATTTTAAAAGCGAGAAGTTAGAGAAGTTTCGATGGAATCTCAAAC
AAGACCTCGGTGACCAGCACACGGAGAAATTCTTAGATTTTACGCAGAAATACT
ATCAAGAGGTCGAGCGTGCTAAGTCTGGAATTTGGGAACAAGAGAGTGCCTCT
TTGCTCGAAAAGGCCGACATAAATCCGCCGTCCAAACGTAAAATACTCAACCG
CTTGGTTGGAAACATCCTGGGAGTAGAGATGTCCGCCGAGGAGCTTACTACAT
TCTTGTCTGATGTTTGGACCAAGAAGGTACACGGTCCATCCACTGTTAAATCTA
TATGTAAGGCAGCTGAGGATTTGCGTAAGCAAAACGGTAATCTGTTTAACATGT
ACATTCAAAGTGTCAGATACCGGGCCTCAGTAGAGGCTGGGGCTGAGAAGAA
GTTCAAGGGCTTAGACAAGGAGATCTGGCAACTGTTAGTGAAGGTTGAAAAGG
CTGCCGACGTTATCAGCGGATCTATCAGCGCAACAGAGACCCAAAAGCAGCGT
TTCTCAAACACATTCAGCTTAGCCCAACTTTATACGTTAATTGAAACCGATCGAA
ATGGCTTTTCCAAAGTGTCGGCAGCAGCGCATATGGAATTAGCTTGGAGAATG
ACTATGATCCCAGTTGATGACAAAGAGGCGGCGCGATGCTCAAGATTACCTGC
TGATTCTTCGAGACCGTTTGATGGTGTTCTTCGCCGTCTGTTGGAGCGACAAG
CATACGAGATTGCAACATTGAAGTCTGGCCAAATTAAGGAAAGCGGCGTGCGC
GATTCCGAGATTTCTATCCCGGTTGTTGTGGAAGAGAATCGTTTCACCTTTAGC
ATCGGGTTAGCCGACGTTAAGAAGGACGGTAGTAAAAGAAAGAAATTACTGAG
TCTTGGAGAAAAGCAGCTCGAAAAGTGGCAGGAGAAGAACGAGAGAATTCGTC
AAGGTGGTAGCGGACTCTGTCCCTACAAAGGGACTATCCTCGACAAGGACGC
GGAACTGGACCATATTATACCGCGTTCTTATTCGCGAGACGCCAGTGGTACTAT

ATTTAACAGTGAGGCGAACCTGATCTATGCAAGTCGTCGCGGAAACCAAGAGA
AGAAGGATCAGAGATATGGAATCTCCGATCTGGCTCCAAACTATCTGAAAGCG
GTGTACGGCACAAGCGACAGTGCTGAAATTACTCGTATCATTGAGGCATCCGT
AGCAAGCATCAAGAAAGATTTTCTGTTTAGTGAATTAGAGGGCGACCAACAGC
GTGACGTACGTCACGCGCTCTTTCTTGAGCGGGACTCTAAAGCGTTCGAGAAA
GTATTTCGTCGGCTTGCGACCCAGCAATCAGCACGCGTTAACGGGACGCAAGC
TTGGCTTGCGCGAAAGCTTATTTCGGTGCTTAAGTCTCAACTCAAGTCATGGCT
TTCCGAGACCGGCAACACAATAGTTTTCAATTTATGCAAGGCCGACGCACAACA
GACCTCTTTTGTCCGTACGGGATTGGCTGTTACCAAACCGGAAACCACTAAGG
GCGAACGGCAAGGCGTGGGCTCACATGCTATTGACGCATACTGCGCCTTTTCT
GTGGCCGCGGCCGAGGGTATGCATGAGACACTGGCACAGGGTGCCGGGCTTA
TGGAAGACACAGAATGGTTAGCTACAGCCCTGCCAGTGGATGTGGACGTCAAC
TGGGTCAAGCGTCTGCCGCTGTACCGAAAATCTGAGCTGTCAACCCGTCCTAT
TTTCAAAGAGGGCATATACGCCGAAAGTTTCTTAACCACATGGATATCGGAAAA
GGGTATAGAGAGCGGCTTTTCTCTGGGCTCAAACAGTGTCCAGTGGCAGGGAA
AAGACACACGAGGTCTGTTAGAGTTGTTGGCTCCGTTTGCTACGAAGAACCTG
AACGTGGACACTGCACTCGCGTCACCGCGTCCGGTGCAATTGGACATATCGAA
GGAGAAGGCATTTAAACATTTTGAGATGGTAGCTAAGGCCCCACACAGTGAAG
TAGACTTACACCAAGCAGACATCCTGGACGCACTGCGCTACTGTACGGCTAAG
AAAGACATTAGCAGCCGATTAATCGACGTGCAAAAGAAGACCCTTAAGAAGCC
TGAAGAGGTTTTAAAAGCGAAGGACATCTATGTTAAAGTGGACGTGGATGTGAA
GGGGCTTGGCAAACTGAAAGGAAAAGTTACCCACCCAGCCATAGAACAGTGGA
AGCGTCTGTTTCTTATTCCAGATATTGAAACCCGTATCGGTGAGAAGGCAGTCG
GTATAAATTGGCAGCAGATAGGCGAGAAGTATTTCGGCGAGGGTAGCAGTCGT
CCCCATTCTAAGACTCGTCGCGTCTACTCACTGCCGATTGTTGACTCTCCAGAT
GGTGGTTACCGCATAAAGCGTATCGCCCCAGACGGCAGTACTATTTGGCAGTT
AACAAGCATTAAGGACACAGGGTCCTCTGGATTCACTGTCAACGATAAAGGGG
TTAACTGGAAACAGCCTGTAGCAATACCGGCCTTACGGGACTCCAGCGTGGTA
GGCGTCGGCGAGCGCTATCAACCGGTGCCGGAACAACAGGTGTATTTTGACG
ACTGGAAGTGTGTTGATATCACAGACGAAAGAGTTCTGAGTGTATCGGTAGCG
CCGGGCACCAAGGACCGACGTTACGTTTCTGTGTCACAAACGCTTTCGGACTT
CAAAGCGTGGCTCACGGAATCCGGCATCAAGCTGCACAGCCCGTTTGAGATAC
CGTCCCGCGTTAAGGTCGACGCCGCCAAGTTTCAAAGCGCCCACGGAATCGAT
GTCCTTGGTAAGCCCAAGAGTAACTTATTCATCTCCAAAGTGGGCGACGTTGTC

AATTACTGGTATATTCGGGAGTCTTCGCCGGTAGTTATAAACCAATACTATGATA
AAGCCTTGCTTGCGGAACGCGAGGCCGTGAATGAAACGGAACCTTGTGATATA
TCTCGTGCGGACCCGAAGAAGAAACGAAAAGTTGCTGCTGCTCTCGAGCACCA
CCACCACCACCACTGA

### Activity in vitro

As a DNA template for testing nuclease activity in vitro, a PCR amplified fragment of 1833 base pairs of the *dpy-10* gene was used. Guide RNAs for nuclease of the invention and FnCas9 were synthetized from a Gblock (IDT) of sequences (SEQ ID NO: 16 y 17, respectively).
SEQ ID NO: 16. Gblock sequence to synthesize DO2 Cas9 gRNA specific for *dpy-10*: 
AAGCTAATACGACTCACTATAGGGCTACCATAGGCACCACGAGGTTTCAGTTG
CAGGTTTATTGGGATAGGGAAACCTATCCCAATAGGCCTGATTGAAGTCGCCC
ACGGGCGAGCTGAAATCATCTAAAATCATGACTGACTCCGGCACCTGCTTCGG
CATCCGGGGTTTTTCTTTTT
SEQ ID NO: 17. Gblock sequence to synthesize FnCas9 Cas9 gRNA specific for *dpy-*10:

AAAGCAAGCAAGCTAATACGACTCACTATAGGGCTACCATAGGCACCACGAGG
TTTCAGTTGCGCCGAAAGGCGCTCTGTAATCATTTAAAAGTATTTTGAACGGAC
CTCTGTTTGACACGTCTG

### 5.2 RESULTS

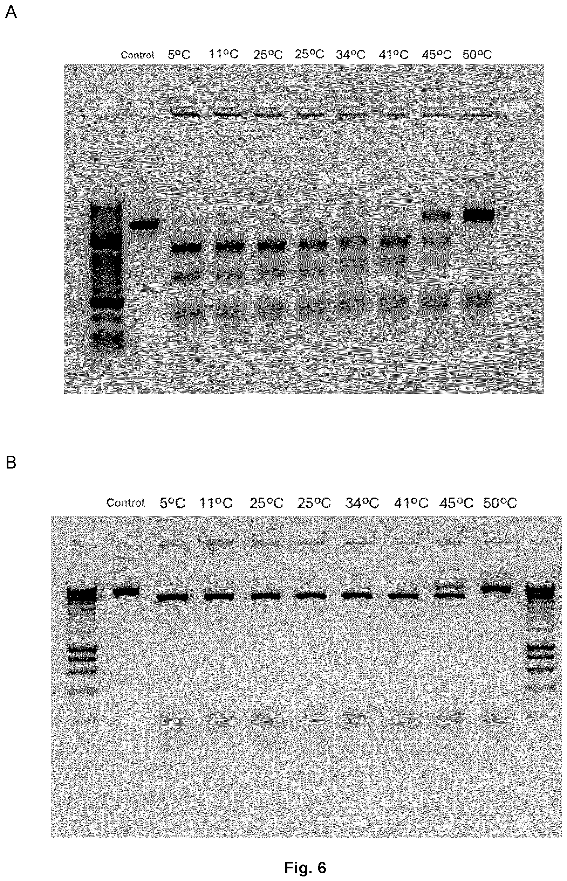

First, the in vitro activity of purified Cas9 DO2 was studied over a temperature range of 5°C to 50°C using two different DNA templates: PCR products to observe bands as a result of cleavage (Figure 6A), and plasmids to observe linearization as a result of cleavage (Figure 6B). Strong in vitro activity was observed between 5°C and 40.9°C, with reduced activity at 45.5°C and inactivation at 50°C.

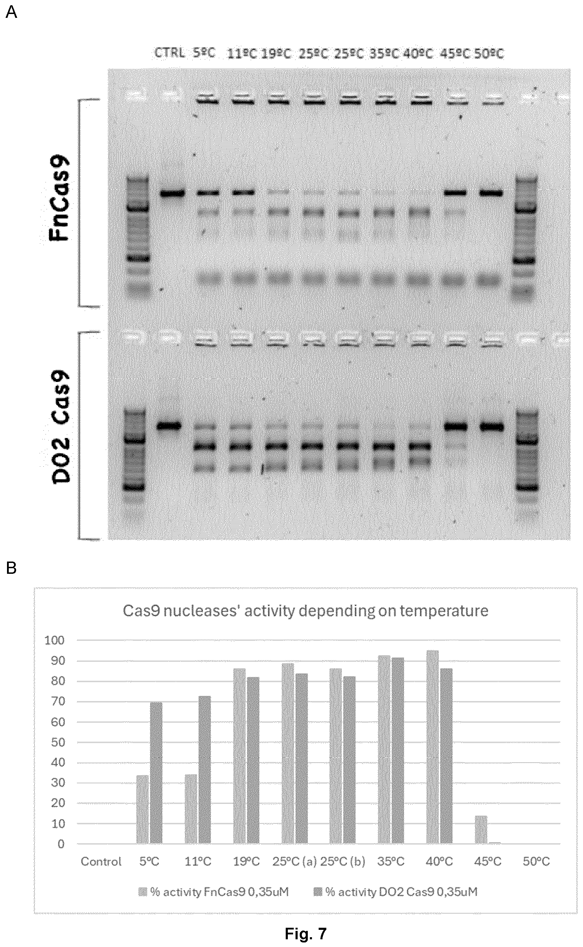

Cas9 DO2 was then compared to its type II-B Cas9 relative FnCas9. Unlike FnCas9, Cas9 DO2 activity is not drastically reduced at lower temperatures such as 5°C or 11°C (Figure 7).

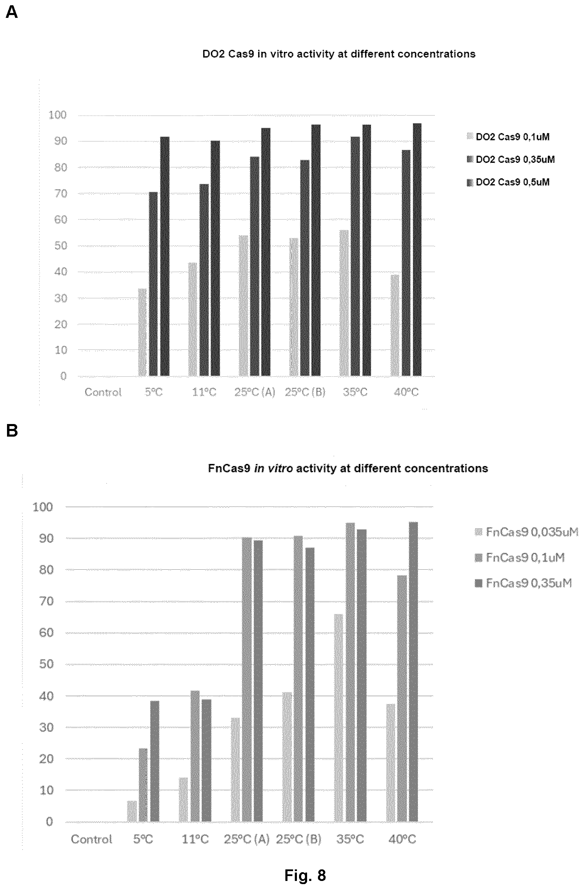

Finally, FnCas9 and DO2 Cas9 were tested at different concentrations and DO2 Cas9 maintained similar in vitro activity from 5°C to 40.9°C (Figure 8A) while FnCas9 activity drops dramatically at 5°C and 15°C at any protein concentration (Figure 8B).

### Example 6. Evaluation of specificity of nuclease determining its tolerance to mismatches

### 6.1 MATERIALS AND METHODS

### 6.1.1 Effects of mismatches in the targeted region on nuclease activity as readout for specificity.

To test the specificity for recognizing mismatches in the target sequence, a sequence-specific guide RNA with a point mutation in the unc-119 gene that produces the *ed3* allele (Maduro& Pilgrim. 1995. Identification and cloning of unc-119, a gene expressed in the Caenorhabditis elegans nervous system. Genetics, 141, 977-88) was used.
SEQ ID NO: 18. with perfect match for the ed3 allele sequence:
   GGCAAGATATGT**CCG**ATATTGATTTGCGCCGAATTTTCTGAAAT
SEQ ID NO: 19 wildtype sequence:
   GGCAAGATATGT**CCG**ATATCGATTTGCGCCGAATTTTCTGAAAT

**Bold** nucleotides correspond to PAM sequence. underscored nucleotides are those related to the point mutations.

For SpCas9 and DO2 Cas9, gRNAs were tested against the target sequence *ed3 unc-119.* These gRNAs were synthesized from the following gblocks:
SEQ ID NO: 20. gBlock gRNA DO2 Cas9 unc-119 (ed3), 5' to 3': 
AAGCTAATACGACTCACTATAGGAATTCGGCGCAAATCAATATGTTTCAGTTGC
AGGTTTATTGGGATAGGGAAACCTATCCCAATAGGCCTGATTGAAGTCGCCCA
CGGGCGAGCTGAAATCATCTAAAATCATGACTGACTCCGGCACCTGCTTCGGC
ATCCGGGGTTTTTCTTTTT
SEQ ID NO: 21. gBlock gRNA for SpCas9 unc-119 (ed3), 5' to 3':

AAGCTAATACGACTCACTATAGGAATTCGGCGCAAATCGATATGTTTTAGAGCT
AGAAATAGCAAGTTAAAATAAGGCTAGTCCGTTATCAACTTGAAAAAGTGGCAC
CGAGTCGGTGCTTTT

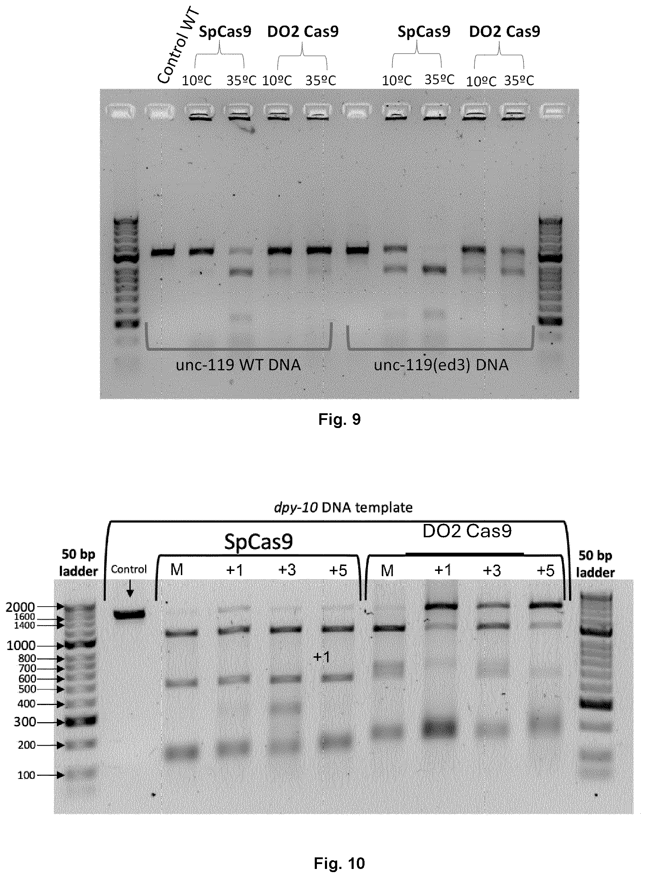

Digestion was performed for 30 minutes at 10°C and 35 °C, using DO2 Cas9 and SpCas9 proteins, and gRNAs, at 0.3 uM (Figure 9).

### 6.1.2. Effects of mismatches in the guide RNA on nuclease activity as readout for specificity.

To further explore the increased specificity of DO2 Cas9 compared to SpCas9, *C*. *elegans dpy-10* guide RNAs with mismatches at positions +1, +3, and +5 were evaluated *in vitro.*

The following gblocks were used to produce control gRNA and mismatched gRNA (shadow and bold nucleotides modified from the original sequence):
SEQ ID NO: 22. *dpy-10* gblock for SpCas9: 
AAGCTAATACGACTCACTATAGGCTACCATAGGCACCACGAGGTTTAAGAGCTA
TGCTGGAAACAGCATAGCAAGTTTAAATAAGGCTAGTCCGTTATCAACTTGAAA
AAGTGGCACCGAGTCGGTGCTTTTTTT
SEQ ID NO: 23. *dpy-10* gblock for SpCas9 with a mismatch at position +1: 
AAGCTAATACGACTCACTATAGGCTACCATAGGCACCACGACGTTTAAGAGCTA
TGCTGGAAACAGCATAGCAAGTTTAAATAAGGCTAGTCCGTTATCAACTTGAAA
AAGTGGCACCGAGTCGGTGCTTTTTTT
SEQ ID NO: 24. *dpy-10* gblock for SpCas9 with a mismatch at position +3: 
AAGCTAATACGACTCACTATAGGCTACCATAGGCACCACAAGGTTTAAGAGCTA
TGCTGGAAACAGCATAGCAAGTTTAAATAAGGCTAGTCCGTTATCAACTTGAAA
AAGTGGCACCGAGTCGGTGCTTTTTTT
SEQ ID NO: 25. *dpy-10* gblock for SpCas9 with a mismatch at position +5: 
AAGCTAATACGACTCACTATAGGCTACCATAGGCACCTCGAGGTTTAAGAGCTA
TGCTGGAAACAGCATAGCAAGTTTAAATAAGGCTAGTCCGTTATCAACTTGAAA
AAGTGGCACCGAGTCGGTGCTTTTTTT
SEQ ID NO: 26. *dpy-10* gblock for DO2 Cas9: 
AAGCTAATACGACTCACTATAGGGCTACCATAGGCACCACGAGGTTTCAGTTG
CAGGTTTATTGGGATAGGGAAACCTATCCCAATAGGCCTGATTGAAGTCGCCC
ACGGGCGAGCTGAAATCATCTAAAATCATGACTGACTCCGGCACCTGCTTCGG
CATCCGGGGTTTTTCTTTTT
SEQ ID NO: 27. *dpy-10* gblock for DO2 Cas9 with a mismatch at position +1: 
AAGCTAATACGACTCACTATAGGGCTACCATAGGCACCACGACGTTTCAGTTGC
AGGTTTATTGGGATAGGGAAACCTATCCCAATAGGCCTGATTGAAGTCGCCCA
CGGGCGAGCTGAAATCATCTAAAATCATGACTGACTCCGGCACCTGCTTCGGC
ATCCGGGGTTTTTCTTTTT
SEQ ID NO: 28. *dpy-10* gblock for DO2 Cas9 with a mismatch at position +3: 
AAGCTAATACGACTCACTATAGGGCTACCATAGGCACCACAAGGTTTCAGTTGC
AGGTTTATTGGGATAGGGAAACCTATCCCAATAGGCCTGATTGAAGTCGCCCA
CGGGCGAGCTGAAATCATCTAAAATCATGACTGACTCCGGCACCTGCTTCGGC
ATCCGGGGTTTTTCTTTTT
SEQ ID NO: 29. *dpy-10* gblock for DO2 Cas9 with a mismatch at position +5:

AAGCTAATACGACTCACTATAGGGCTACCATAGGCACCTCGAGGTTTCAGTTGC
AGGTTTATTGGGATAGGGAAACCTATCCCAATAGGCCTGATTGAAGTCGCCCA
CGGGCGAGCTGAAATCATCTAAAATCATGACTGACTCCGGCACCTGCTTCGGC
ATCCGGGGTTTTTCTTTTT

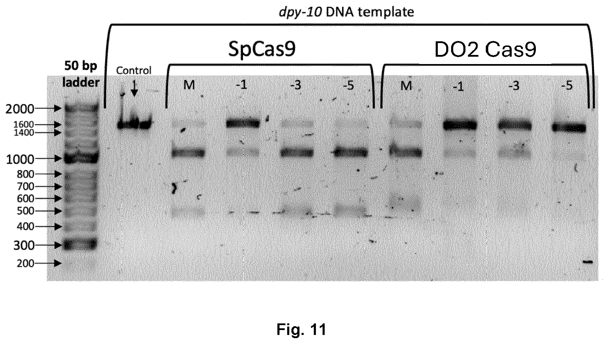

Conditions for RNP formation and digestion temperature were the same for both SpCas9 and DO2 Cas9. The stock for *dpy-10* DNA template was at 93 ng/ul, and the final concentration of template in each reaction was set to 6.33 ng/uL. The control reaction contained nuclease-free water, BlueJuice and the DNA template. The RNP formation was done at 37°C (Figure 10) or 20°C (Figure 11) for 30 minutes. The DNA template was incubated for 5 min at its digestion temperature (37°C or 20°C) or prior to its addition to the RNP complex. Digestion (RNP + template) was for 30 minutes. Then, added 1uL of Proteinase K (20 mg/mL) to each digestion and let them incubate at 56°C for 10 min. Then, added 3uL of BlueJuice to each digestion and let incubate for 10 minutes at 70°C. The different reactions were run in a 1.5% agarose electrophoresis gel at 110V for 20 minutes.

### 6.2 RESULTS

### 6.2.1. DO2 Cas9 display lower activity than SpCas9 in the presence of a mismatch in the target sequence

As expected, SpCas9 and DO2 Cas9 cut the DNA template of *unc-119 (ed3)* (right side in Figure 9 at both temperatures, although SpCas9 activity is more affected by the temperature drop to 10°C. Interestingly, SpCas9 also cut at 35°C in the WT template containing a mismatch whereas DO2 Cas9 at the same temperature show a minimal activity (Figure 9). This result suggests that DO2 Cas9 would be more specific than SpCas9.

### 6.2.2. DO2 Cas9 display lower activity than SpCas9 in the presence of mismatches in the guide RNA

Either a 37°C (Figure 10) or 20°C (Figure 11), DO2 Cas9 shows similar efficacy to cut the target DNA when using a gRNA without mismatches but less activity in the presence of mismatches indicating higher specificity.

### Example 7. Evaluation of the thermosensitivity and other biophysics properties of DO2 Cas9 nuclease.

### 7.1 MATERIALS AND METHODS

### 7.1.1 Determination of temperatures affecting the activity of DO2 Cas9 and class II-B family member FnCas9 exposed to heat before and after RNP formation.

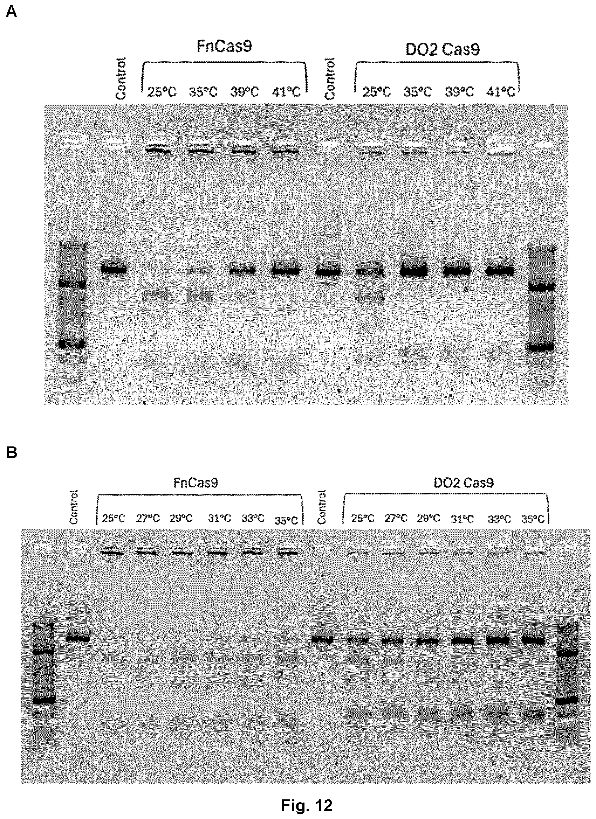

The thermosensitivity of DO2 Cas9 and FnCas9 without gRNA was evaluated. These proteins were tested in a thermocycler at different temperatures. In an initial experiment, DO2 Cas9 and FnCas9, previous to RNP formation, were exposed to 25°C, 35°C, 39°C and 41°C (Figure 12) and later to 25°C, 27°C, 29°C, 31°C, 33°C, 35°C. After challenging proteins at different temperatures, all reactions were frozen at 20°C for 90 minutes. RNPs formation was induced at 25°C for 30 minutes. Then DNA template was added, and digestion took place at 25°C for 30 minutes, using protein concentration of 0.35µM and gRNA at 0.35µM. Reaction was inactivating by adding 3 µl of bluejuice and incubated at 70°C for 10 minutes.

### 7.1.2 Determination of thermal stability, melting point, and activation energy of nuclease using circular dichroism (CD) and fluorescence spectroscopies (FS).

**Circular dichroism** monitors the ellipticity of the protein when the sample is heated at a constant rate (1°C per minute). The physical observable is related to the amount of secondary structure of the protein. At low temperatures, the protein being in its folded state, has a large negative ellipticity indicative of a well-packed conformation in which the different elements of secondary structure interact thermodynamically favourably stabilizing the native (fully active) conformation. The increase in temperature would induce the (partial) unfolding of the protein and, therefore, the loss of some elements of its secondary structure.

The unfolding process is only complete for small globular proteins in very defined conditions (acidic pH, low salt concentration, etc.). For most proteins, the initial unfolding of the protein leads to the population of partially folded conformations with the simultaneous exposure to the solvent of large patches of apolar groups (previously buried in the native conformation). As a result, these partially folded states tend to agglomerate together to avoid the hydration of the exposed hydrophobic patches leading to the precipitation of the denatured protein.

Thus, the experiments were done with protein concentrations in the interval 0.18 - 0.22 mg.mL-1 in HEPES buffer supplemented with 200 mM NaCl at pH 7.5. The ellipticity of the protein was monitored from 6 to 80°C (the interval 6 - 70°C is represented for clarity) and the sample was heated at a constant gradient of 1°C. min-1.

The experimentally determined ellipticity vs. temperature data was fitted to a sigmoidal Boltzman equation assuming linear initial and final baselines (for either native or denatured states). Two main features are relevant: the inflection point of the sigmoidal (the temperature at which the curve turns from concave to convex) that is usually denoted by tₘ and the slope of the sigmoidal curve t = tₘ , that is inversely related to dt: a low value of dt denotes a large slope (the sigmoidal is less open and the transition between the two conformational states is completed in a smaller temperature interval). On the contrary, a large value of dt means that the slope at tm is small, the sigmoidal curve looks more spread and the complete transformation of the native into the denatured state needs a larger temperature interval to be completed.

On the other hand, **fluorescence spectroscopy** monitors the intrinsic emission of (mainly) tryptophan (TRP) residues upon excitation at a given wavelength. Both the position (maximum wavelength) and intensity of the emission band depend strongly upon the hydration (degree of exposure of these residues to the aqueous solvent). Therefore, by following the heat-induced change in the fluorescence emission of a protein, the local environment around the TRP residues was monitored. The intrinsic fluorescence of the protein reveals changes in the solvent accessibility of TRP. The denaturation of the protein may be detected following these changes in the fluorescence intensity of the protein as a function of temperature, but the overall process might be obscured when the degree of TRP residues exposure to the solvent is similar in the initial (native) and final state (aggregated), leading to a spectroscopically silent process.

Protein concentrations were in the interval 0.05 - 0.07 mg mL-1 in HEPES buffer supplemented with 200 mM NaCl at pH 7.5. The ellipticity of the protein was monitored from 15 to 80⁰C (the interval 15 - 70°C is represented for clarity) and the sample was heated at constant gradient of 1°C min-1. DO2 Cas9 was dialyzed before the experimental study. To compare with CD studies, experiments were carried out using a fresh frozen stock.

### 7.2 RESULTS

### 7.2.1 Determination of the exposure to temperature necessary to inactivate DO2 Cas9 and FnCas9 nucleases

DO2 Cas9 is denatured after one hour at 33°C whereas FnCas9 was more resistant to temperature, showing activity even after one hour at 35°C (Figure 12).

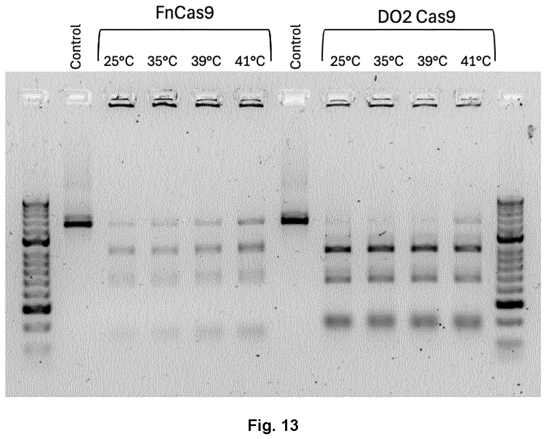

However, DO2 Cas9 as RNP is active after being challenged to 39°C for one hour (Figure 13).

### 7.2.2 Determination of thermal stability and activation energy of nuclease using circular dichroism (CD) and fluorescence spectroscopies (FS) for DO2 Cas9, SpCas9, and FnCas9

The initial results obtained by CD and FS clearly indicated that the heat-induced denaturation of the proteins was irreversible leading to aggregation.

### Results from DC

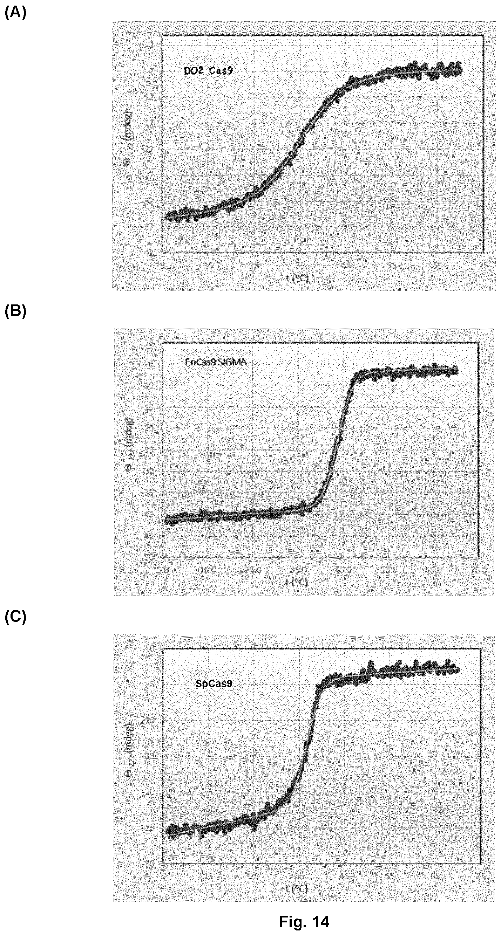

The ellipticity of the protein remains fairly stable (increases slightly, in negative values) while the protein is fully populating a given conformational state. As the sample absorbs increasing amounts of energy in the form of heat, the protein starts to unfold, losing some elements of secondary structure leading to an increase in its ellipticity in a sigmoidal fashion until the final (aggregated) state is fully populated. The sigmoidal behavior is indicative that the kinetically controlled process involves mainly only two states: the initial native state and the final denatured one (Figure 14).

DO2 Cas9 thermal stability as determined by tm is much lower than that of FnCas9. However, it is like SpCas9 tm. However, regarding dt, the differences indicate that DO2 Cas9 has an activation energy of the heat-induced denaturation process different from FnCas9 and SpCas9 (Table 6).

For SpCas9, its dt value (1.7) is low meaning that the activation energy is high, and the rate of denaturation increases rapidly with temperature. Denaturation of SpCas9 seems to start slightly below 25°C and is completed when temperature reaches 45°C.

In the case of DO2 Cas9, its dt is higher (4.7) meaning that the activation energy is low, and denaturation transition seems to start at temperatures slightly above 25°C and increase gradually instead of rapidly.

**Table 6. Analysis of thermal denaturation of proteins tested monitored using circular dichroism.**

| | Circular dichroism | | | |
|---|---|---|---|---|
| | tm (°C) | | dt | |
| Protein | Value | Error | Value | Error |
| SpCas9 | 36.8 | 0.5 | 1.7 | 0.3 |
| Nuclease of the invention | 35.4 | 0.3 | **4.7** | 0.5 |
| FnCas9 (SIGMA) | 43.8 | 0.4 | 1.6 | 0.2 |

### Results from FS

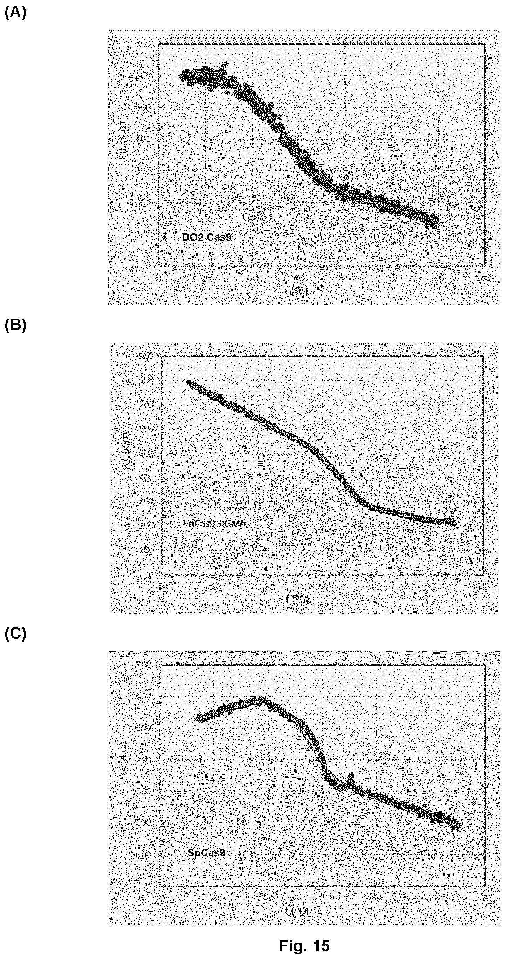

Similar results and conclusions are obtained when analyzing the temperature dependence of the fluorescence intensity of the proteins. Figure 15 presents the results of the thermal denaturation experiments using fluorescence.

The experimentally determined fluorescence intensity vs. temperature data was fitted to a sigmoidal Boltzman equation assuming linear initial and final baselines (for either native or denatured states). This equation highly improves the reproducibility of the fit (especially the two relevant parameters, tm and dt) when different wavelengths are monitored for a single experiment (Table 7).

Considering that the variation of fluorescence intensity with temperature would only give the changes in hydration in the local environment of the TRP residue, it was considered more reliable the results obtained from the CD studies. Nevertheless, it is important to note that the fluorescence results are qualitatively in line with the ones discussed before regarding the circular dichroism experiments.

**Table 7. Analysis of temperature dependence of the fluorescence intensity of the proteins.**

| | Steady-state fluorescence | | | |
|---|---|---|---|---|
| | tm (°C) | | dt | |
| Protein | Value | Error | Value | Error |
| Nuclease of the invention | 38.0 | 0.8 | **6.7** | 0.7 |
| SpCas9 | 35.9 (*) | 1.0 | 2.7 (*) | 0.8 |

| | | | | |
|---|---|---|---|---|
| *(*): Values could be affected by the increase in the turbidity of the sample (and therefore on the dispersion of emitted light) due to massive aggregation of the protein upon its denaturation* | | | | |

### Example 8. Evaluation in vivo nuclease activity in Caenorhabditis elegans

### 8.1 MATERIALS AND METHODS

### 8.1.1. Evaluation nuclease genome editing in Caenorhabditis elegans

Young adult *Caenorhabditis elegans* hermaphrodites at the L4 stage were immobilized on 2% agar pads with halocarbon oil and injected into the germline with the corresponding transformation mix using the XenoWorks Microinjection System (Sutter Instrument) and the Nikon Eclipse Ti-S inverted microscope with Nomarski optics. The final reaction volume was 10 µL, and the mixture was incubated at 20 °C for 15 minutes. Afterward, it was centrifuged at 13,000 rpm for 2 minutes to allow the settling of particulate matter. The injection mixes were kept on ice before loading the needles, and any excess was stored at -20°C. The injected days. The F1 progeny were then separated individually or in pools onto NGM agar plates, allowed to lay F2 progeny, and subsequently genotyped via PCR.

### CRISPR mix with protein:

1ul DO2 Cas9 (3.3uM); 0.5ul gRNA synthetized from gBlock (3.2 uM); and 2ul *mlc-*1:GFP plasmid (130ng/uL); and nuclease-free water to complete 10 ul.

### 8.1.2. Evaluation of nuclease sensitivity to mismatches in gRNA in vivo in Caenorhabditis elegans.

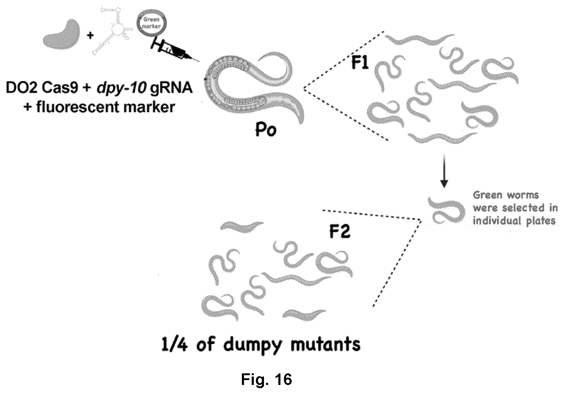

The experiment depicted in Figure 16 was performed with SpCas9 and DO2 Cas9 using perfect guide RNAs and guide RNAs with mismatches at position +3.

### 8.2. RESULTS

### 8.2.1. Nuclease genome editing in Caenorhabditis elegans.

Worm from the progeny (F1) showing fluorescence (from *mlc*-*1*:GFP) were singled-out. Out of 45 animals showing GFP fluorescence (marker for an effective injection), 27 display a *dpy-10* mutant phenotype either in F1 or F2 generation, representing a 60% of editing efficiency in vivo. F2 dumpy mutants were selected for DNA extraction and amplification of the *dpy-10* gene (Figure 16). PCR amplicons for *dpy-10* gene in dumpy mutants were sequenced (Figure 16).

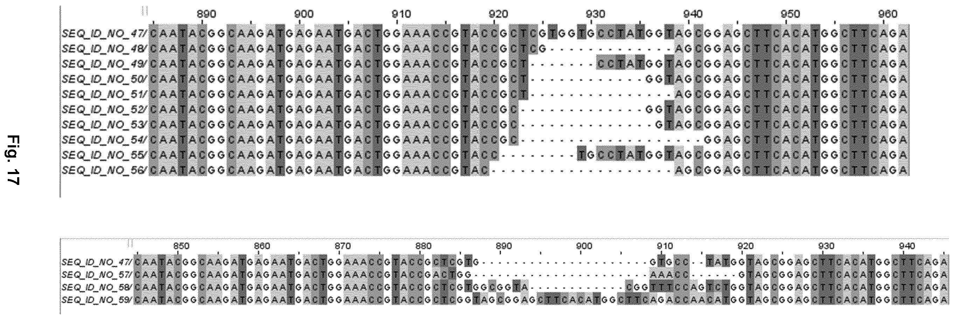

By doing Sanger sequencing on PCR fragments of *C*. *elegans dpy-10* mutants produced by DO2 Cas9, indels (insertions or deletions) compatible with the DO2 Cas9 cutting site were found at position -3 (target DNA strand) and -5, -6 (non-target DNA strand) (SEQ ID NO: 47 for wild-type sequence) (SEQ ID NO: 48 to 59 for representative edits produced by DO2 Cas9 on *dpy-10* gene) (Figure 17).

### 8.2.2. Nuclease sensitivity to mismatches in gRNA in vivo in Caenorhabditis elegans.

As a result, SpCas9 (n = 102 green animals) and DO2 Cas9 (n = 29 green animals) showed 98% and 44.8% efficiency, respectively. However, using guide RNAs with a mismatch at position +3 (previously used in vitro, Figures 9 and 10), SpCas9 (n = 154 green animals) and DO2 Cas9 (n = 30 green animals) showed 43.5% and 0% efficiency, respectively, indicating higher specificity for DO2 Cas9.

### Example 9. Evaluation in vivo nuclease activity in Zebrafish

### 9.1 MATERIALS AND METHODS

DO2 Cas9 mRNA (225 pg) and DO2 gRNA2 (150 pg) against the slc45a2 zebrafish gene were injected in zebrafish embryos, and then incubated a 25°C and 33°C.

Besides using mRNA to express DO2 Cas9 in zebrafish embryos, purified protein was used as RNP.

DO2 Cas9 gblocks used for gRNAs synthesis:
SEQ ID NO: 30. gRNA slc45a2-g: 
TAATACGACTCACTATAGGGGAAGGTTGATTATGCACGTTTCAGTTGCAGGTT
TATTCCCTATCCCTTTGGATAGGGTTATCCGGACTAACTTCAGCGGGTGCCC
GCTCGACTTTAGTAGATTTTAGTACTGACTGAGGCCGTGGACGAAGCCGTAG
GCCCCAAAAAGAAAAA
SEQ ID NO: 31. gRNA slc45a2-f: 
TAATACGACTCACTATAGGAGCCTCCGAGGCGCTCTAGTTTCAGTTGCAGGT
TTATTCCCTATCCCTTTGGATAGGGTTATCCGGACTAACTTCAGCGGGTGCC
CGCTCGACTTTAGTAGATTTTAGTACTGACTGAGGCCGTGGACGAAGCCGTA
GGCCCCAAAAAGAAAAA
SEQ ID NO: 32. gRNA slc45a2-k:

TAATACGACTCACTATAGGGTCCGTCAATGAAGTCTGGTTTCAGTTGCAGGTT
TATTCCCTATCCCTTTGGATAGGGTTATCCGGACTAACTTCAGCGGGTGCCC
GCTCGACTTTAGTAGATTTTAGTACTGACTGAGGCCGTGGACGAAGCCGTAG
GCCCCAAAAAGAAAAA

### 9.2 RESULTS

Editing efficiency reaches about 70% in one case whereas editing was not detected in the case of another gRNA (Figure 17). This experiment suggests that DO2 Cas9 would be more efficient in vivo at 33°C than at 25°C.

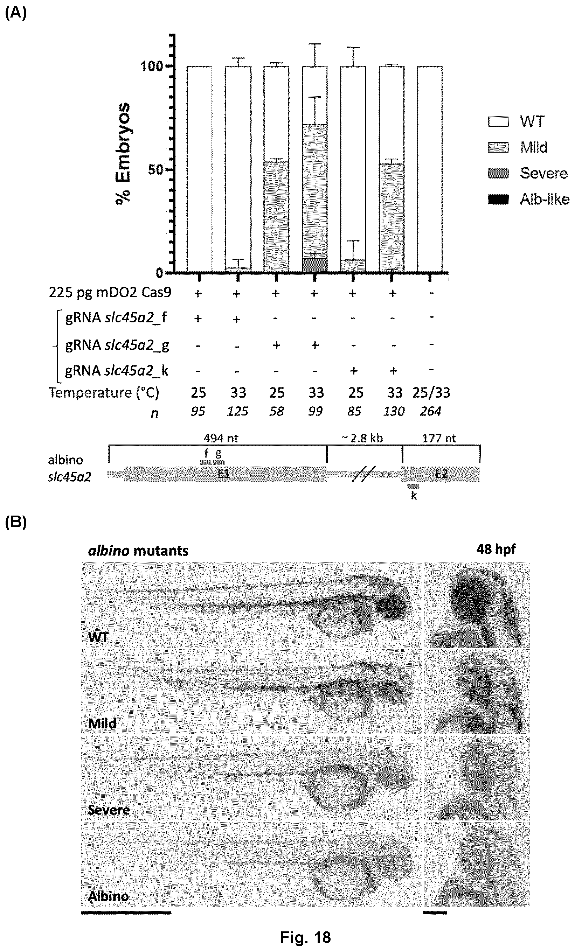

Using purified protein as RNP was also efficient in gene editing, although the protein concentration would be optimized for greater efficiency (Figure 18).

### Example 10. Evaluation in vivo nuclease activity in plants (Nicotiana benthamiana)

### 10.1 MATERIALS AND METHODS

*Nicotiana benthamiana* leaves were co-transfected with two Agrobacterium tumefaciens strains, one expressing DO2 Cas9 protein and other expressing DO2 Cas9 gRNA.

For determining the in vivo editing capacity of DO2 Cas9 in plant cells, *Nicotiana benthamiana* leaves were transiently co-transformed with three gene constructs, the first of them SEQ ID NO: 33 containing a constant transcriptional unit (TU1) encoding the DO2 Cas9 open reading frame, the second (SEQ ID NO: 34 or SEQ ID NO: 35) containing a variable TU2 expressing the corresponding guide RNA and a third one (SEQ ID NO: 36) containing a constant transcriptional unit (TU3) encoding the silencing suppressor of the Tomato Bushy Stunt Virus P19.

The TU1 comprised a constitutive promoter double CaMV35S, followed by a *Nicotiana benthamiana* codon-optimized DO2 Cas9 ORF, and the CaMV35S terminator. The DO2 Cas9 ORF was interrupted by eight Arabidopsis introns located at regular positions within the DO2 Cas9 CDS.

The TU2 comprised an *Arabidopsis thaliana* U6-26 promoter, followed by a variable 20 nucleotides protospacer sequence and a DO2 Cas9 -specific constant scaffold sequence terminated by a polyT sequence.

The TU3 comprised a constitutive promoter CaMV35S, followed by the P19 ORF, and the terminator of the Nopaline synthase gene.

TU1, TU2 and TU3 are cloned within a pCambia binary plasmid, flanked by right and left border sequences for Agrobacterium-mediated transient transformation. The 20-nucleotides protospacer (SEQ ID NO: 37) in the gene construct described in SEQ ID NO: 34 was directed against the exon 1 of the *Nicotiana benthamiana* phytoene desaturase homologue genes NbL03g24820.1 and NbL13g18760.1
SEQ ID NO: 37. The 20-nucleotides protospacer in the gene construct described in SEQ ID NO: 34:
TTGGTAGTAGCGACTCCATG

The 20-nucleotides protospacer (SEQ ID NO: 38) in the gene construct described in SEQ ID NO: 35 was directed against the exon 3 of the *Nicotiana benthamiana* magnesium-chelatase subunit ChIH homologue genes NbL05g17570.1 and NbL10g22050.1.
SEQ ID NO: 38. The 20-nucleotides protospacer in the gene construct described in SEQ ID NO: 35:
CTTCTTGCTAAAGCTAACAG

The respective binary plasmids were transformed into Agrobacterium tumefaciens strain EHA105; the three strains carrying TU1, TU2 an TU3 respectively were mixed in equivalent amounts and syringe-agroinfiltrated into *Nicotiana benthamiana* leaves.

Five days post-infiltration, agroinfiltrated leaves were harvested and their genomic DNA extracted. Next, DNA regions surrounding the genomic targeted sites were PCR amplified for each targeted gene using the specific oligonucleotides SEQ ID NO: 39 and SEQ ID NO: 40 for NbL03g24820.1; SEQ ID NO: 41 and SEQ ID NO: 42 for NbL13g18760.1; SEQ ID NO: 43 and SEQ ID NO: 44 for NbL05g17570.1, and SEQ ID NO: 45 and SEQ ID NO: 46 for NbL10g22050.1.

The following are described as follows DNA regions surrounding the genomic targeted sites were PCR amplified for each targeted gene using the specific oligonucleotides:
SEQ ID NO: 39.
   GGCTTAATTTACTGCTATCTTGTTCAA
SEQ ID NO: 40.
   CAGTTAATCAGAAAAGAATGTACAGG
SEQ ID NO: 41.
   GGCTTAATTTACTGCTATTTTGTTCAG
SEQ ID NO: 42.
   CCAAAACAGTTAATAAGAAAAGAATGC
SEQ ID NO: 43.
   TTACGACACGAAGTATATAG
SEQ ID NO: 44.
   AACTCGAAGATCATGAGCAG
SEQ ID NO: 45.
   TTACGATAGGAAGTATATTT
SEQ ID NO: 46.
   AACTCGAAGATCATGATCTA

### 10.2 RESULTS

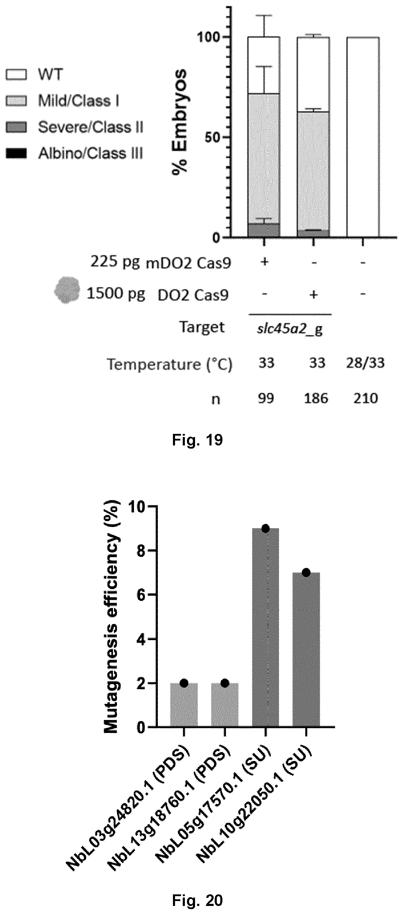

As result of transitory expression of both plasmids, an editing efficiency of 9% was observed maintaining the plants at temperature of 22-24°C (Figure 20).

The resulting PCR amplicons were subsequently Sanger-sequenced and the resulting chromatograms were analyzed for peak heterogenicity indicative of the presence of somatic mutations in the targeted areas, resulting from DO2 Cas9 nuclease activity. The analysis of the chromatograms using Synthego software was employed to quantify the proportion of mutated sequences in each amplicon. For both phytoene desaturase homologue targets, Synthego software estimated a 2% mutagenesis efficiency. For the two magnesium-chelatase homologue targets, Synthego software estimated 9% and 7% mutagenesis efficiency respectively.

These results indicate the DO2 Cas9 nuclease is active as a targeted mutagenesis agent in the plant cell.

### Example 11. Determination of structure of nuclease and potential versions resulting from catalytic key residues.

### 9.1 MATERIALS AND METHODS

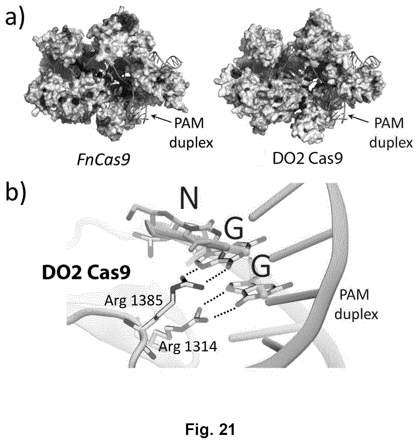

To determine and/or observe the overall similarity of FnCas9 and DO2 Cas9 (Figure 21A) and identify residues relevant for catalytic activity and PAM recognition (Figure 21B), AlphaFold *(*Jumper et al. Highly accurate protein structure prediction with AlphaFold. Nature. 2021: 596(7873), 583-589) was used as a modeling tool.

### 9.2 RESULTS

DO2 Cas9 has a potential capacity to bind and degrade ssRNA (due to similarities to structural FnCas9), which means potential as diagnostic tools and in the protection against ssRNA viruses.

## Claims

1. A ribonucleoprotein complex comprising:
(i) a nuclease, wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1; and
(ii) a guide RNA, wherein the guide RNA is
- A dual guide RNA comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3, and
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4;
or
- A single guide RNA comprising a nucleotide sequence having a sequence identity of at least 70%, at least 75%, at least 80%, at least 85%, at least 90%, at least 95%, at least 98%, at least 99% or 100% to SEQ ID NO: 5.

2. The ribonucleoprotein complex according to claim 1, wherein the nuclease binds and cleaves the target polynucleotide in a protospacer adjacent motif (PAM) - dependent manner.

3. The ribonucleoprotein complex according to claim 1 or 2, wherein the nuclease cleaves:
- a double-stranded target polynucleotide, at the +3 position from PAM in the target DNA strand and at the -5 and -6 position from PAM in the non-target strand, leaving mostly overhangs of 2 or 3 nucleotides,
or
- a single-stranded target polynucleotide.

4. The ribonucleoprotein complex according to claim 2 or 3, wherein the PAM sequence comprises the sequence NGG wherein N is any base and G is Guanine.

5. The ribonucleoprotein complex according to any one of claims 1 to 4, wherein the nuclease as ribonucleoprotein complex is enzymatically active in a temperature range from 5°C to 45.5°C.

6. The ribonucleoprotein according to any one of claims 1 to 5, wherein the nuclease as ribonucleoprotein complex is enzymatically inactive in a temperature above 45.5°C.

7. A dual guide RNA comprising:
a) a CRISPR RNA (crRNA) comprising a nucleotide sequence having a sequence identity of, at least 70%, at least 75%, at least 80%, at least 85%, at least 90%, at least 95%, at least 98%, at least 99% or 100% to SEQ ID NO: 3,
b) a transactivating crRNA (tracrRNA) comprising a nucleotide sequence having a sequence identity of at least 70%, at least 75%, at least 80%, at least 85%, at least 90%, at least 95%, at least 98%, at least 99% or 100% to SEQ ID NO: 4.

8. A single guide RNA comprising a nucleotide sequence having a sequence identity of at least 70%, at least 75%, at least 80%, at least 85%, at least 90%, at least 95%, at least 98%, at least 99% or 100% to SEQ ID NO: 5.

9. A vector comprising
- a polynucleotide comprising a nucleotide sequence encoding a nuclease, wherein the nuclease comprises an amino acid sequence having a sequence identity of at least 70%, at least 75%, at least 80%, at least 85%, at least 90%, at least 95%, at least 98%, at least 99% or 100% to SEQ ID NO: 1, preferably, the nucleotide sequence encoding the nuclease comprises the sequence SEQ ID NO: 2; and/or
- a dual guide RNA According to claim 7; and/or
- a single guide RNA according to claim 8.

10. A cell comprising:
(i) the ribonucleoprotein complex according to any one of claims 1 to 6, and/or
(ii) a nuclease comprising an amino acid sequence having a sequence identity of at least 70%, at least 75%, at least 80%, at least 85%, at least 90%, at least 95%, at least 98%, at least 99% or 100% to SEQ ID NO: 1, and/or
(iii) a polynucleotide comprising a nucleotide sequence encoding the nuclease according to (ii), and/or
(iv) the dual guide according to claim 7, and/or
(v) the single guide according to claim 8, and/or
(vi) the vector according to claim 9.

11. A nuclease comprising an amino acid sequence having a sequence identity of at least 70%, at least 75%, at least 80%, at least 85%, at least 90%, at least 95%, at least 98%, at least 99% or 100% to SEQ ID NO: 1.

12. A polynucleotide comprising a nucleotide sequence encoding a nuclease according to claim 11, preferably, the nucleotide sequence encoding the nuclease comprises the sequence SEQ ID NO: 2.

13. Use of
(i) the ribonucleoprotein complex according to any one of claims 1 to 6, and/or
(ii) the dual guide according to claim 7, and/or
(iii) the single guide according to claim 8, and/or
(iv) the vector according to claim 9, and/or
(v) the nuclease according to claim 11, and/or
(vi) the polynucleotide according to claim 12,
for modifying *in vitro* a target nucleic acid sequence that comprises binding, cutting or cleaving specifically a target nucleic acid sequence, wherein the elements (i) to (vi) are not use for modifying the germ line genetic identity of human beings.

14. Use of the ribonucleoprotein complex, the dual guide RNA, the single guide RNA, the vector, the nuclease or the polynucleotide according to claim 13, wherein cutting or cleavage of a target nucleic acid sequence is carried out in a temperature range of from 5°C to 45.5°C.

15. A ribonucleoprotein complex according to any one of claims 1 to 6, and/or a dual guide according to claim 7, and/or a single guide according to claim 8, and/or a vector according to claim 9, and/or a nuclease according to claim 11, and/or a polynucleotide according to claim 12, for use in therapeutic modifying a target nucleic acid sequence that comprises binding, cutting or cleaving specifically a target nucleic acid sequence.
